# EUROPEAN PATENT APPLICATION

(11) **EP 1 747 769 A2**
(43) Date of publication of application: **31.01.2007**
(21) Application number: 06253553.9
(22) Date of filing: 06.07.2006
(51) Int. Cl.: A61F 2/16

(54) **Intraocular devices**

(30) Priority: 26.07.2005 US 191180
(71) Applicant: Visioncare Ophthalmic Technologies, Inc., Saratoga, California 95070 (US)
(72) Inventor: Aharoni, Eli, Tel Aviv (IL); Gross, Yossi, Moshav Mazor (IL)
(74) Representative: Draper, Martyn John

(57) **Abstract**

An intraocular device including at least one lens (182) and a flexible integrally formed support element (160) for retaining the at least one lens, the support element including first and second axially separated anchor portions (168,170) which are joined by at least one flexible joining element (164) which supports the at least one lens without having to be fixed thereto.

## Description

### FIELD OF THE INVENTION

The present invention relates to intraocular implants generally and more particularly to implantable implant devices and implantation methodologies.

### BACKGROUND OF THE INVENTION

The following U.S. Patents and published patent applications of applicant/assignee are believed to represent the current state of the art:

U.S. Patents 4,074,368; 4,172,297; 4,373,218; 4,759,761; 4,892,543; 4,976,732 4,994,082; 5,222,981; 5,384,606; 5,391,202; 5,628,798; 5,653,751; 5,769,889; 5,769,890; 5,814,103; 5,876,442; 5,928,283; 6,007,579; 6,066,171; 6,464,725; and 6,596,026;

Published U.S. Applications 2001/018,612; 2004/117,011; and 2004/138,746.

The following patent publications are also believed to be of interest:

Published PCT Applications WO 94/07,435; WO 00/38593 and WO 83/01566;

Foreign Patent Publications DE 4,403,326; EP 1,092,402; EP 0,419,740; GB 2,181,355; EP 0,897,702; EP 0,212,616; DE 3,428,895 and DE 19,501,444.

### SUMMARY OF THE INVENTION

The present invention seeks to provide improved structures and installation methodologies for intraocular implants.

There is thus provided in accordance with a preferred embodiment of the present invention an intraocular device including at least one lens and a flexible integrally formed support element for retaining the at least one lens, the support element including first and second axially separated anchor portions which are joined by at least one flexible joining element which supports the at least one lens without having to be fixed thereto.

Preferably, the support element is adapted to be anchored to the eye at a ciliary sulcus thereof by engagement of the first and second anchor portions with the ciliary sulcus. Additionally or alternatively, the support element is operative to retain the at least one lens from undesired axial motion along a central axis defined by an iris of the eye.

There is also provided in accordance with another preferred embodiment of the present invention an intraocular device including at least one optical lens and at least one flexible elongate support element arranged to support the at least one optical lens within an eye, without being fixed to the at least one optical lens.

Preferably, the at least one flexible elongate support element is adapted to be anchored to the eye at one of a ciliary body and a ciliary sulcus thereof. Alternatively or additionally, the at least one flexible elongate support element includes multiple flexible elongate support elements defining a retaining structure.

Preferably, the at least one flexible elongate support element is operative to retain the at least one optical lens against undesired motion toward a rear of the eye. Additionally or alternatively, the at least one flexible elongate support element is operative to retain the at least one optical lens from undesired axial motion along a central axis defined by an iris of the eye. Alternatively or additionally, the at least one flexible elongate support element is operative to retain the at least one optical lens from undesired radial motion relative to a central axis defined by an iris of the eye.

Preferably, the at least one optical lens has haptics fixed thereto.

There is further provided in accordance with yet another preferred embodiment of the present invention an intraocular device including at least one optical lens and an anterior chamber locatable lens retainer removably engageable with the at least one optical lens when located in an anterior chamber of an eye for restricting generally axial displacement of the at least one optical lens in a rearward direction away from the anterior chamber.

Preferably, the at least one optical lens has haptics fixed thereto. Additionally or alternatively, the anterior chamber locatable lens retainer engages the optical lens in a snap-fit type engagement. In accordance with another preferred embodiment the anterior chamber locatable lens retainer includes an inner portion for engaging the optical lens and an outer portion for retaining the anterior chamber locatable lens retainer against an iris of an eye, preventing the anterior chamber locatable lens retainer from motion rearward of the iris.

Preferably, the lens retainer includes at least one broadened support portion. Additionally, the at least one broadened support portion is adapted to be seated within a canal between the iris and a cornea of the eye.

There is yet further provided in accordance with still another preferred embodiment of the present invention an intraocular device support including an integrally formed support element having first and second axially separated anchor portions which are joined by at least one flexible joining element which allows the axial separation of the first and second axially separated anchor portions to be varied by axial compression thereof.

Preferably, the support element is adapted to be anchored to an eye at a ciliary sulcus thereof by engagement of the first and second anchor portions with the ciliary sulcus.

There is still further provided in accordance with another preferred embodiment of the present invention a method for implanting an intraocular device including providing at least one optical lens and positioning within an eye at least one flexible elongate support element arranged to support the at least one optical lens within the eye, without being fixed to the at least one optical lens.

Preferably, the method for implanting an intraocular device also includes drawing the at least one flexible elongate support element through the eye at one of the ciliary body and ciliary sulcus. Additionally or alternatively, the positioning includes positioning within an eye multiple flexible elongate support elements defining a retaining structure.

There is also provided in accordance with yet another preferred embodiment of the present invention a method for implanting an intraocular device including inserting and compressing a support element into an anchored orientation within an eye and thereafter inserting at least one lens into supported engagement with the support element in the anchored orientation within the eye.

Preferably, the inserting and compressing includes inserting a first anchor portion into supported engagement with the eye, compressing the support element to provide clearance for a second anchor portion to be inserted into the eye and inserting the second anchor portion into supported engagement with the eye. Additionally, the compressing causes movement of the second anchor portion relative to the first anchor portion.

Preferably, the method for implanting an intraocular device also includes inserting at least one guide into the eye prior to the inserting and compressing. Preferably, the at least one guide includes two guides located parallel to each other. Additionally, the two guides are located on opposite sides of a central axis defined by an iris of the eye.

There is even further provided in accordance with still another preferred embodiment of the present invention a method for implanting an intraocular device including providing at least one optical lens and in an anterior chamber of an eye, employing a lens retainer to engage the at least one optical lens, restricting generally axial displacement of the at least one optical lens in a rearward direction away from the anterior chamber.

Preferably, the retainer engages the optical lens in a snap-fit engagement.

### BRIEF DESCRIPTION OF THE DRAWINGS

The present invention will be understood and appreciated more fully from the following detailed description taken in conjunction with the drawings in which:

Figs. 1A, 1B & 1C are respective pictorial, side sectional and front view illustrations of a first stage of implantation of an implantable intraocular device in accordance with a preferred embodiment of the present invention;

Figs. 2A, 2B & 2C are respective pictorial, side sectional and front view illustrations of a second stage of implantation of an implantable intraocular device in accordance with a preferred embodiment of the present invention;

Figs. 3A, 3B & 3C are respective pictorial, side sectional and front view illustrations of a third stage of implantation of an implantable intraocular device in accordance with a preferred embodiment of the present invention;

Figs. 4A, 4B & 4C are respective pictorial, side sectional and front view illustrations of a fourth stage of implantation of an implantable intraocular device in accordance with a preferred embodiment of the present invention;

Figs. 5A, 5B & 5C are respective pictorial, side sectional and front view illustrations of a fifth stage of implantation of an implantable intraocular device in accordance with a preferred embodiment of the present invention;

Figs. 6A, 6B & 6C are respective pictorial, side sectional and front view illustrations of a final stage of implantation of an implantable intraocular device in accordance with a preferred embodiment of the present invention;

Figs. 7A, 7B & 7C are respective pictorial, side sectional and front view illustrations of a first stage of implantation of an implantable intraocular device in accordance with another preferred embodiment of the present invention;

Figs. 8A, 8B & 8C are respective pictorial, side sectional and front view illustrations of a second stage of implantation of an implantable intraocular device in accordance with another preferred embodiment of the present invention;

Figs. 9A, 9B & 9C are respective pictorial, side sectional and front view illustrations of a third stage of implantation of an implantable intraocular device in accordance with another preferred embodiment of the present invention;

Figs. 10A, 10B & 10C are respective pictorial, side sectional and front view illustrations of a fourth stage of implantation of an implantable intraocular device in accordance with another preferred embodiment of the present invention;

Figs. 11A, 11B & 11C are respective pictorial, side sectional and front view illustrations of a fifth stage of implantation of an implantable intraocular device in accordance with another preferred embodiment of the present invention;

Figs. 12A, 12B & 12C are respective pictorial, side sectional and front view illustrations of a final stage of implantation of an implantable intraocular device in accordance with another preferred embodiment of the present invention;

Figs. 13A, 13B & 13C are respective pictorial, side sectional and front view illustrations of a first stage of implantation of an implantable intraocular device in accordance with yet another preferred embodiment of the present invention;

Figs. 14A, 14B & 14C are respective pictorial, side sectional and front view illustrations of the intraocular optical device of Figs. 13A-13C inserted into the posterior chamber of the eye;

Figs. 15A, 15B & 15C are respective pictorial, side sectional and front view illustrations of a retaining clip prepared for placement within the anterior chamber of the eye in accordance with a preferred embodiment of the present invention;

Figs. 16A, 16B & 16C are respective pictorial, side sectional and front view illustrations of the retaining clip of Figs. 15A-15C placed into the anterior chamber of the eye in engagement with the intraocular optical device of Figs. 13A-14C;

Figs. 17A, 17B & 17C are respective pictorial, side sectional and front view illustrations of a retaining clip prepared for placement within the anterior chamber of the eye in accordance with another preferred embodiment of the present invention;

Figs. 18A and 18B are respective side sectional and front view illustrations of a first step in the insertion of the retaining clip of Figs. 17A-17C into the anterior chamber of the eye in engagement with the intraocular optical device of Figs. 13A-14C;

Figs. 19A and 19B are respective side sectional and front view illustrations of a further step in the insertion of the retaining clip of Figs. 17A-17C into the anterior chamber of the eye in engagement with the intraocular optical device of Figs. 13A-14C;

Figs. 20A and 20B are respective side sectional and front view illustrations of a final step in the insertion of the retaining clip of Figs. 17A-17C into the anterior chamber of the eye in engagement with the intraocular optical device of Figs. 13A-14C;

Figs. 21A, 21B & 21C are respective pictorial, side sectional and front view illustrations of the retaining clip of Figs. 17A-20B placed into the anterior chamber of the eye in engagement with the intraocular optical device of Figs. 13A-14C;

Figs. 22A, 22B & 22C are respective pictorial, side sectional and front view illustrations of a retaining clip prepared for placement within the anterior chamber of the eye in accordance with yet another preferred embodiment of the present invention;

Figs. 23A and 23B are respective side sectional and front view illustrations of a first step in the insertion of the retaining clip of Figs. 22A-22C into the anterior chamber of the eye in engagement with the intraocular optical device of Figs. 13A-14C;

Figs. 24A and 24B are respective side sectional and front view illustrations of a further step in the insertion of the retaining clip of Figs. 22A-22C into the anterior chamber of the eye in engagement with the intraocular optical device of Figs. 13A-14C; and

Figs. 25A, 25B & 25C are respective pictorial, side sectional and front view illustrations of the retaining clip of Figs. 22A-24B placed into the anterior chamber of the eye in engagement with the intraocular optical device of Figs. 13A-14C.

### DETAILED DESCRIPTION OF PREFERRED EMBODIMENTS

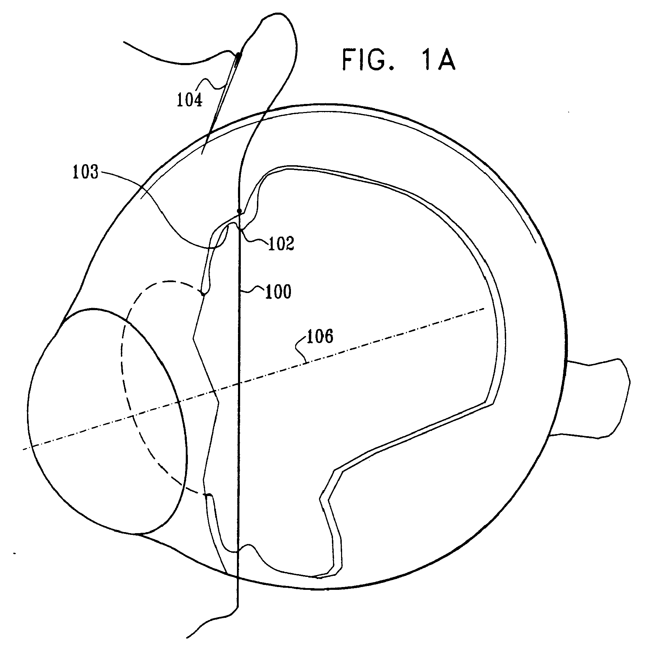

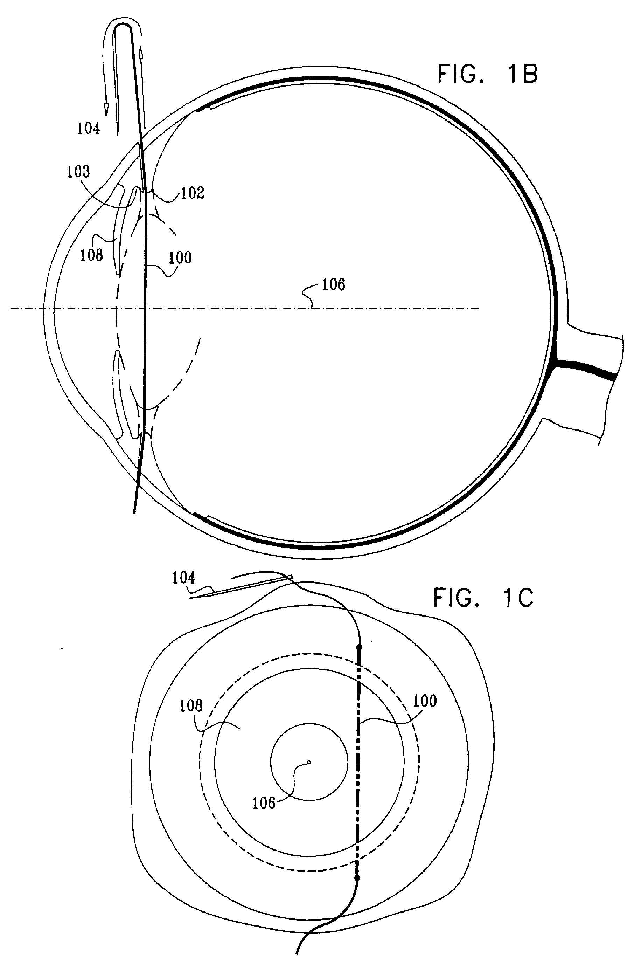

Reference is now made to Figs. 1A, 1B & 1C, which are respective pictorial, side sectional and front view illustrations of a first stage of implantation of an implantable intraocular device in accordance with a preferred embodiment of the present invention. As seen in Figs. 1A - 1C, typically following removal of the lens capsule from the eye, a first flexible elongate support element 100, preferably a portion of thread or suture, is drawn in a straight line through the eye at the ciliary body 102, typically by means of a needle 104 manipulated by a surgeon. As seen particularly in Fig. 1C, the first flexible elongate support element 100 is preferably positioned to one side of a central axis 106 defined by the iris 108. Alternatively, first flexible elongate support element 100 is drawn through the eye at the ciliary sulcus 103 or any other suitable location.

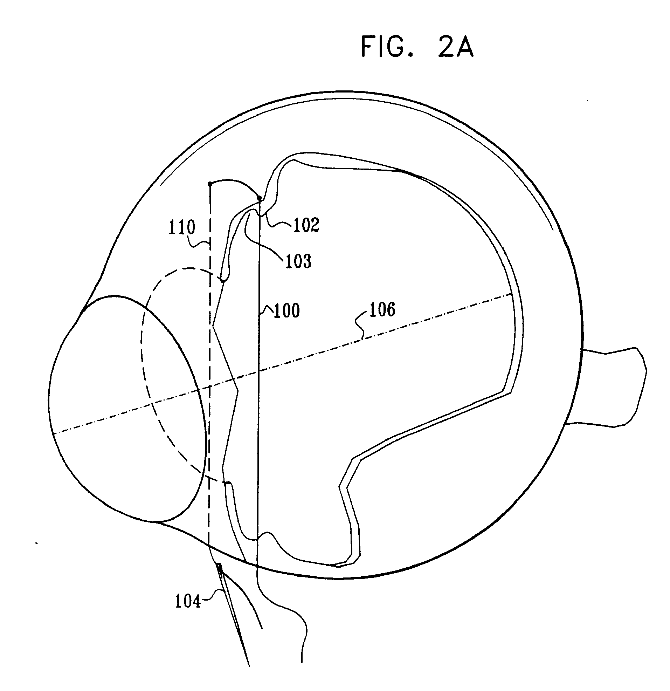

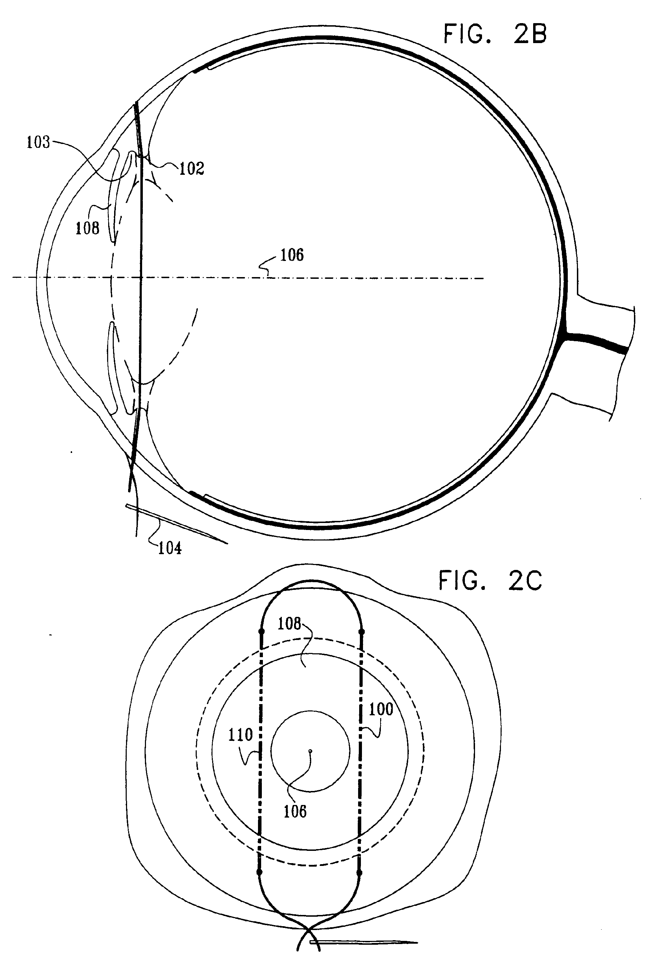

Turning to Figs. 2A, 2B & 2C, it is seen that subsequently a second flexible elongate support element 110, preferably a portion of thread or suture, which may be integral with first flexible elongate support element 100, is drawn in a straight line through the eye at the ciliary body 102, parallel to and spaced from the first flexible elongate support element 100 and preferably coplanar therewith in a plane which is perpendicular to axis 106. As seen particularly in Fig. 2C, second flexible elongate support element 110 preferably lies on an opposite side of central axis 106 from first flexible elongate support element 100. Alternatively, second flexible elongate support element 110 is drawn through the eye at the ciliary sulcus 103 or any other suitable location.

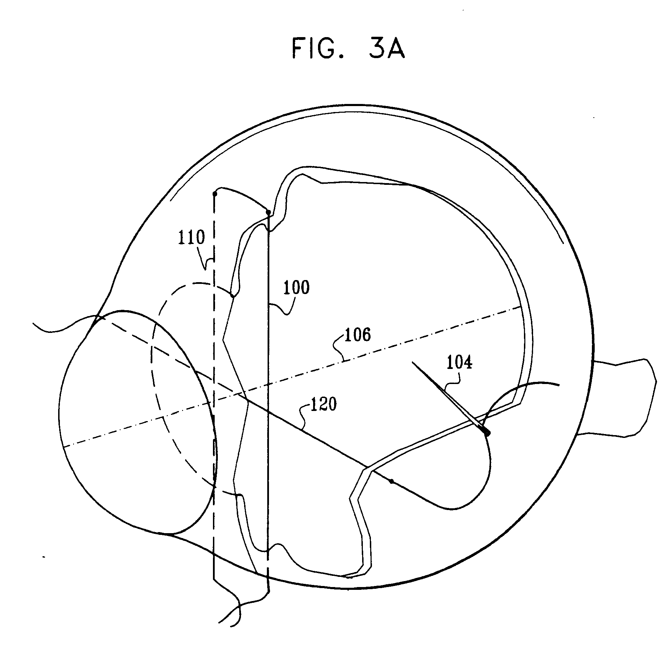

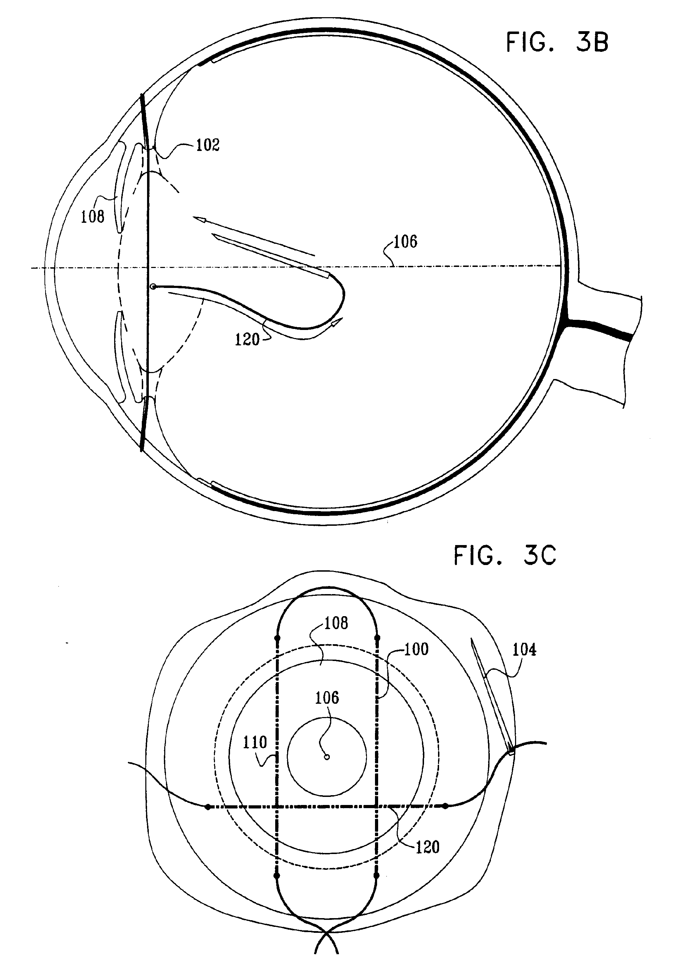

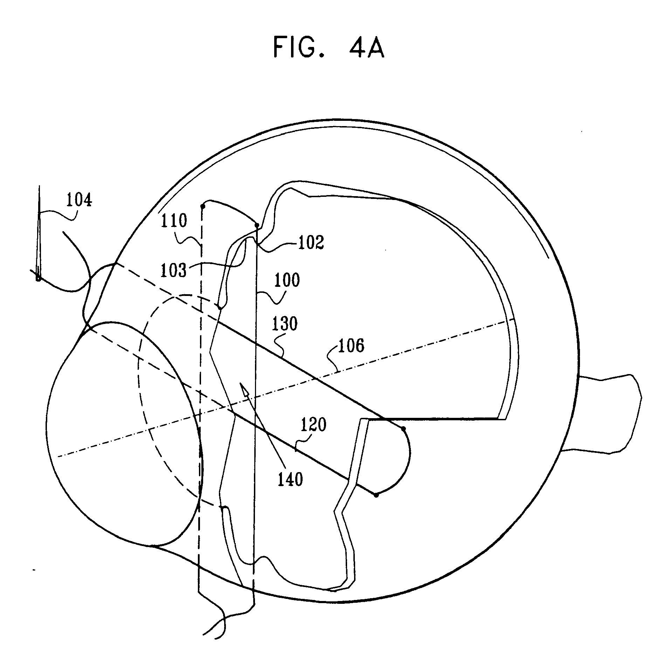

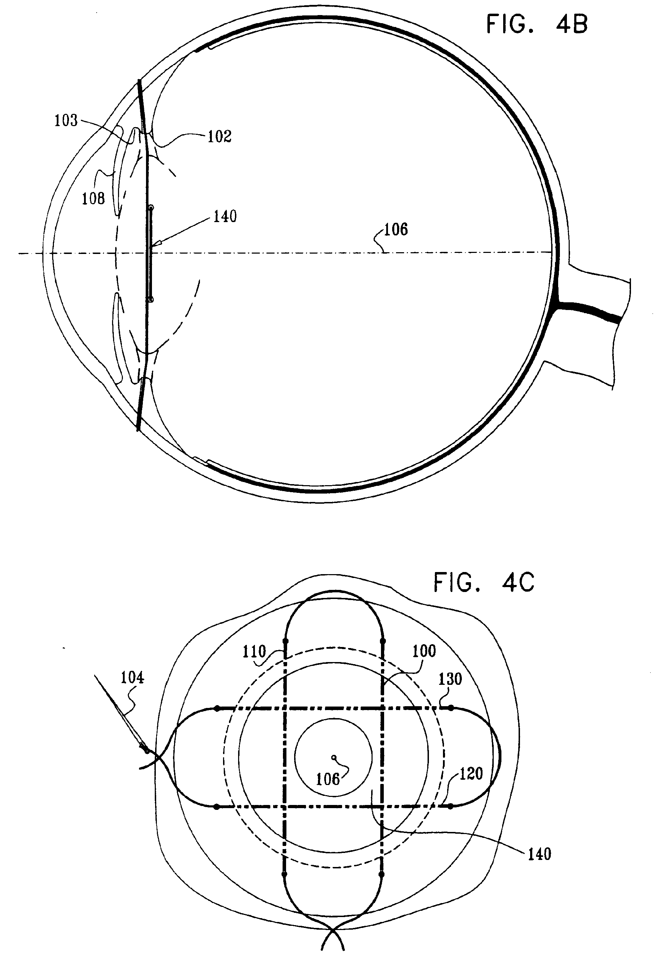

Figs. 3A - 3C and Figs. 4A - 4C respectively illustrate the placement in the eye of respective third and fourth flexible elongate support elements 120 and 130, each preferably a portion of thread or suture, in mutually spaced orientation generally coplanar with flexible elongate support elements 100 and 110 but along lines perpendicular thereto.

It is appreciated that mutually intersecting flexible elongate support elements 100, 110, 120 and 130 define a retaining structure 140 within the eye. Preferably the retaining structure 140 is formed at the ciliary body 102. Alternatively, retaining structure 140 is formed at the ciliary sulcus 103 or other suitable location. The dimensions of the retaining structure are selected in accordance with the dimensions of an intraocular device which is sought to be implanted. Typically, the retaining structure 140 forms a square.

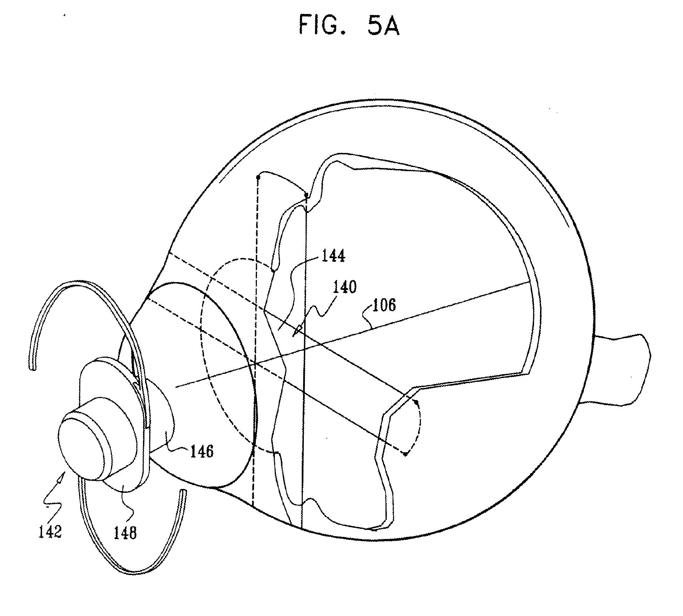

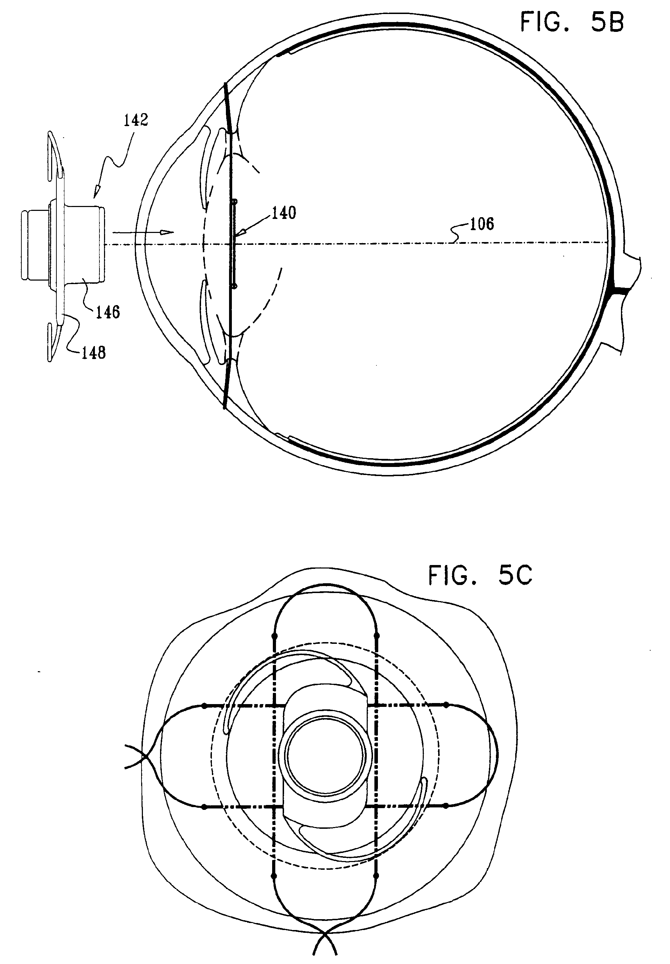

Turning now to Figs. 5A, 5B & 5C, it is seen that a suitable intraocular optical device 142, such as a device described in any of the U.S. Patents or Published U.S. Patent applications listed hereinabove in the Background of the Invention, the disclosures of which are hereby incorporated by reference, is to be inserted into the eye and held by the retaining structure 140 in an orientation which is centered along axis 106 and retained against undesired axial motion along axis 106, particularly motion towards the rear of the eye. Alternatively, there may be applications where maintenance of a centered orientation is not required.

It is appreciated that retaining structure 140 defines an opening 144, which is sufficiently large to allow a generally elongate portion 146 of intraocular optical device 142 to extend therethrough, but is sufficiently small so as to prevent a generally planar portion 148 of the device 142 from extending therebeyond and so as to retain device 142 in a centered position about axis 106.

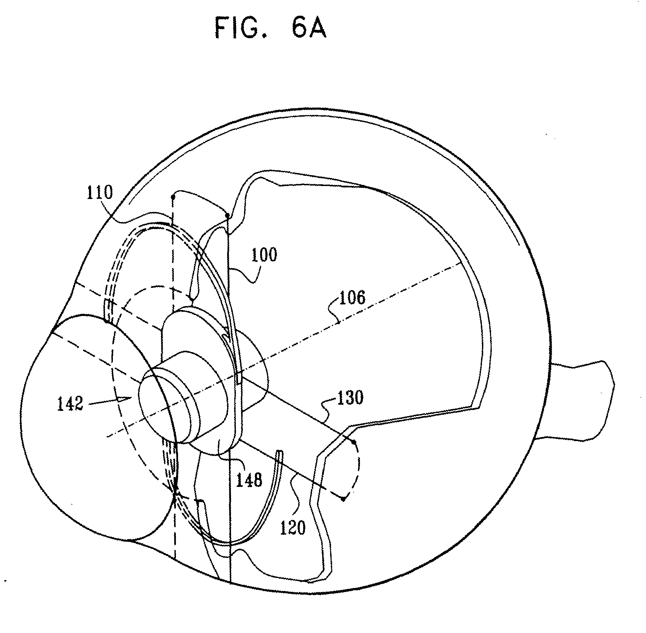

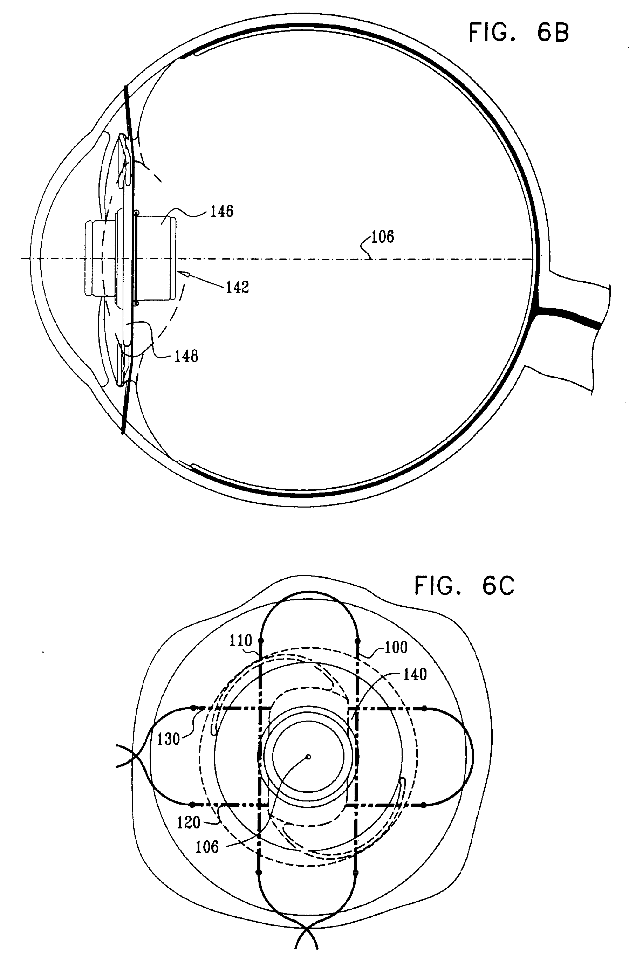

Reference is now made to Figs. 6A, 6B & 6C which illustrate the intraocular optical device 142 implanted within the eye and being centered and retained against undesired rearward movement along axis 106 by engagement of planar portion 148 thereof with retaining structure 140 defined by flexible elongate support elements 100, 110, 120 and 130.

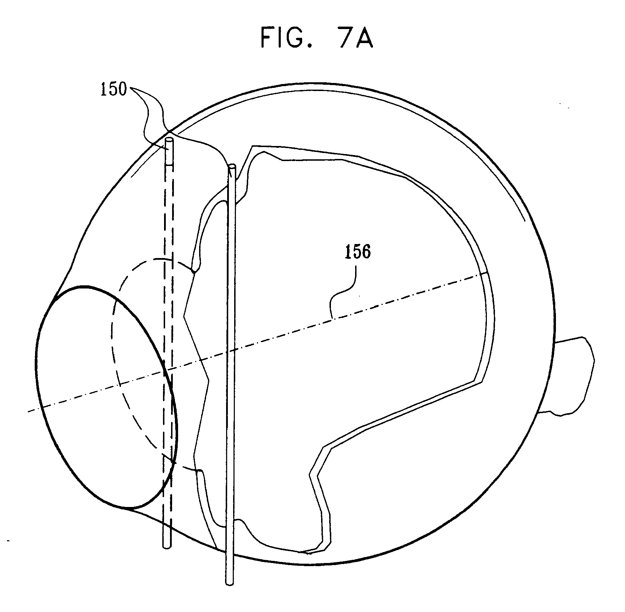

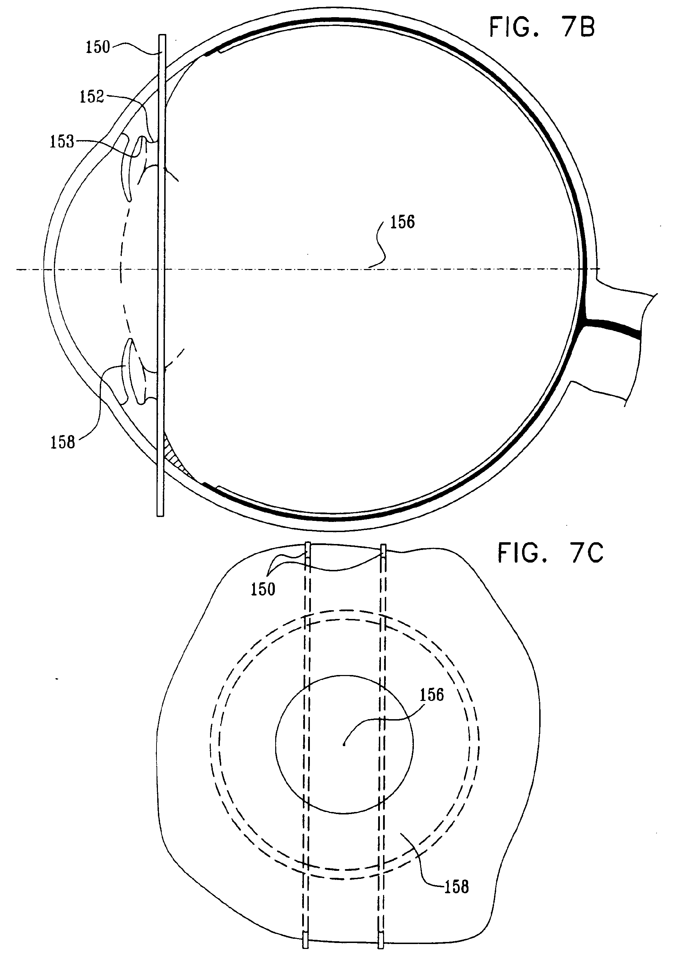

Reference is now made to Figs. 7A, 7B & 7C, which are respective pictorial, side sectional and front view illustrations of a first stage of implantation of an implantable intraocular device in accordance with another preferred embodiment of the present invention. As seen in Figs. 7A-7C, two elongate guides 150, such as needles, are inserted through the eye such that the ends of guides 150 extend beyond the outer wall of the eye. As seen particularly in Fig. 7B, guides 150 are preferably positioned at the ciliary body 152 directly behind the ciliary sulcus 153. As seen particularly in Fig. 7C, guides 150 are preferably located parallel to each other on opposite sides of a central axis 156 defined by the iris 158. Guides 150 provide for insertion and proper placement into the eye of an optics support element, as described further hereinbelow.

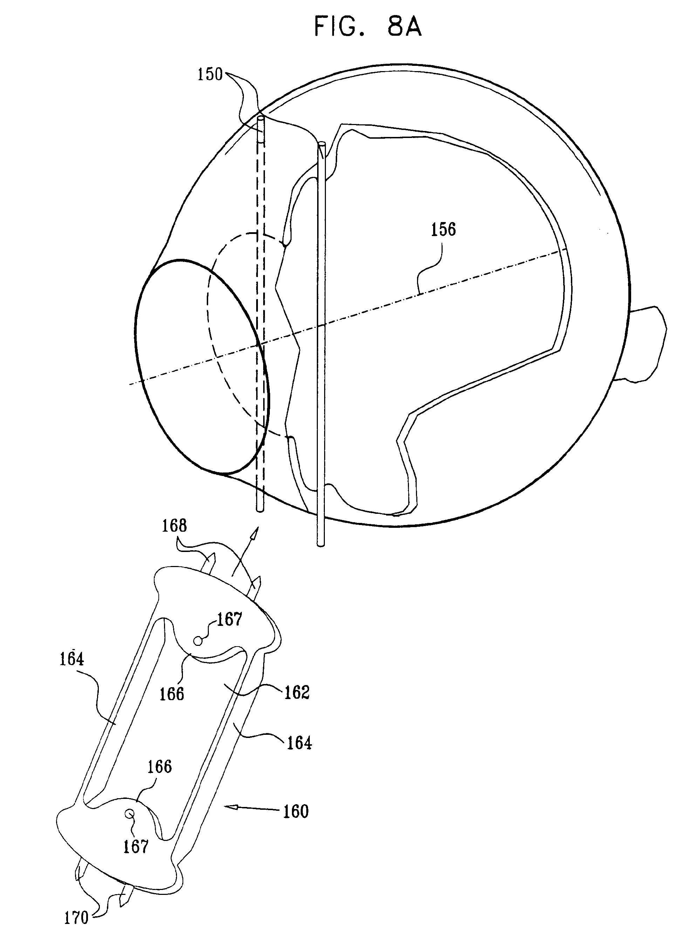

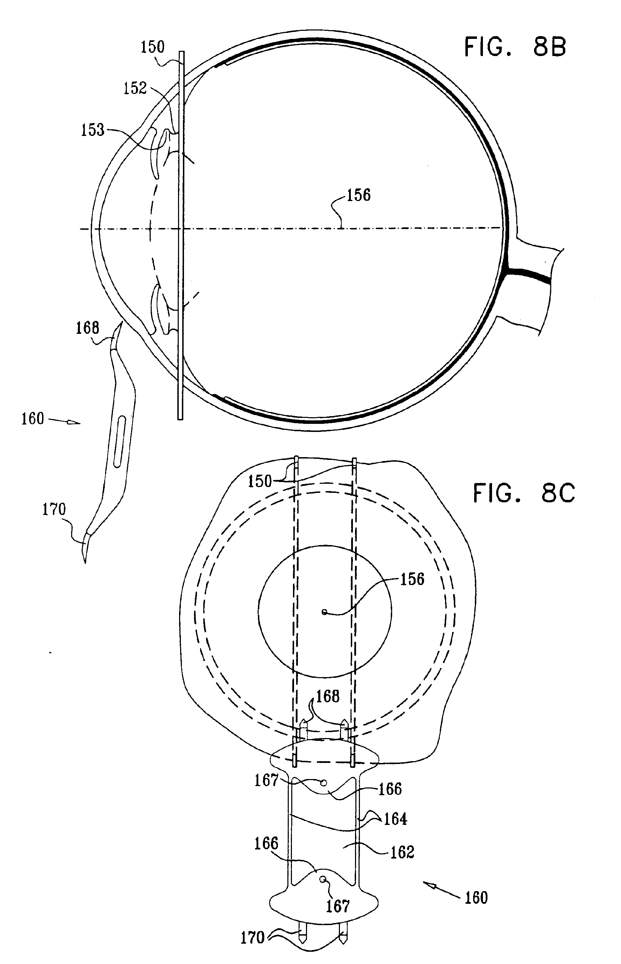

Reference is now made to Figs. 8A, 8B & 8C, which are respective pictorial, side sectional and front view illustrations of a support element 160 prepared for insertion into the eye along guides 150 of Figs. 7A-7C. Support element 160 is preferably top-to-bottom and side-to side symmetrical and defines an optics receiving opening 162. Support element 160 is preferably integrally formed of a flexible, resilient plastic, such as PMMA, and comprises at least one flexible joining element. In the illustrated embodiment the at least one flexible joining element includes flexible resilient side portions 164. Support element 160 also includes compression engagement portions 166, each including hook insertion aperture 167. Support element 160 preferably also comprises outwardly extending first and second anchor portions 168 and 170.

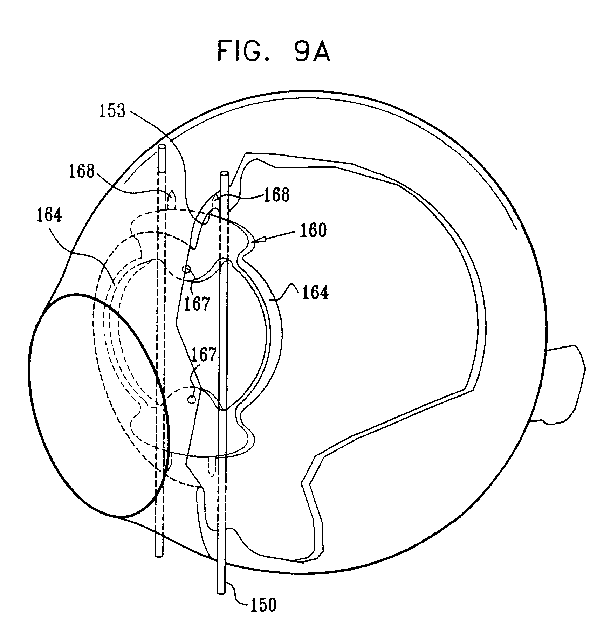

It is appreciated that flexible, resilient, side portions 164 enable support element 160 to be compressed as it is placed in the eye, and then to return to its original shape, as described hereinbelow with reference to Figs. 9A-10C.

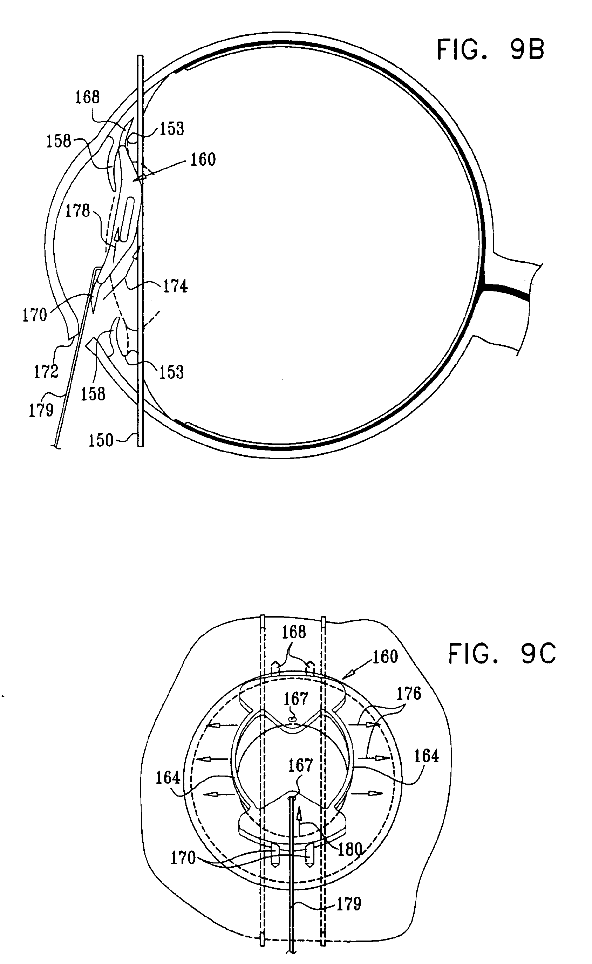

Reference is now made to Figs. 9A, 9B & 9C, which are pictorial, side sectional and front view illustrations of a first stage of placement of support element 160 into the eye through an opening 172 in the cornea.

As seen particularly in Fig. 9B, support element 160 is moved along guides 150, in an upward direction relative to the eye, as shown by arrow 174, into a position where first anchor portions 168 engage the ciliary sulcus 153. Further movement of support element 160 upwardly along guides 150 causes side portions 164 to bend outwardly, as shown particularly by arrows 176 in Fig. 9C. The outward bending of side portions 164 moves second anchor portions 170 relative to first anchor portions 168, as shown by arrow 178 in Fig. 9B, and provides ample clearance to allow ends of second anchor portions 170 to be placed into the eye without interference from the iris 158. In accordance with a preferred embodiment of the present invention, the compression of support element 160 is achieved by inserting a hook 179 into hook insertion aperture 167 adjacent anchor portions 170 and pushing or pulling hook 179 in the direction of first anchor portions 168, as shown by arrow 180 in Fig. 9C.

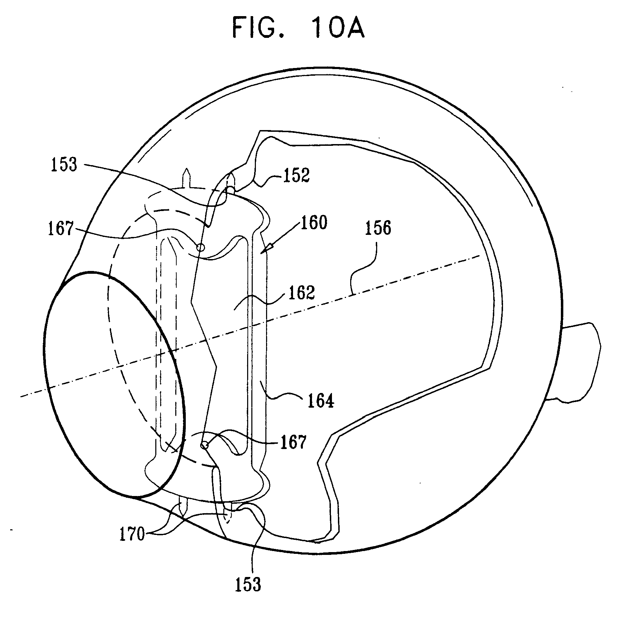

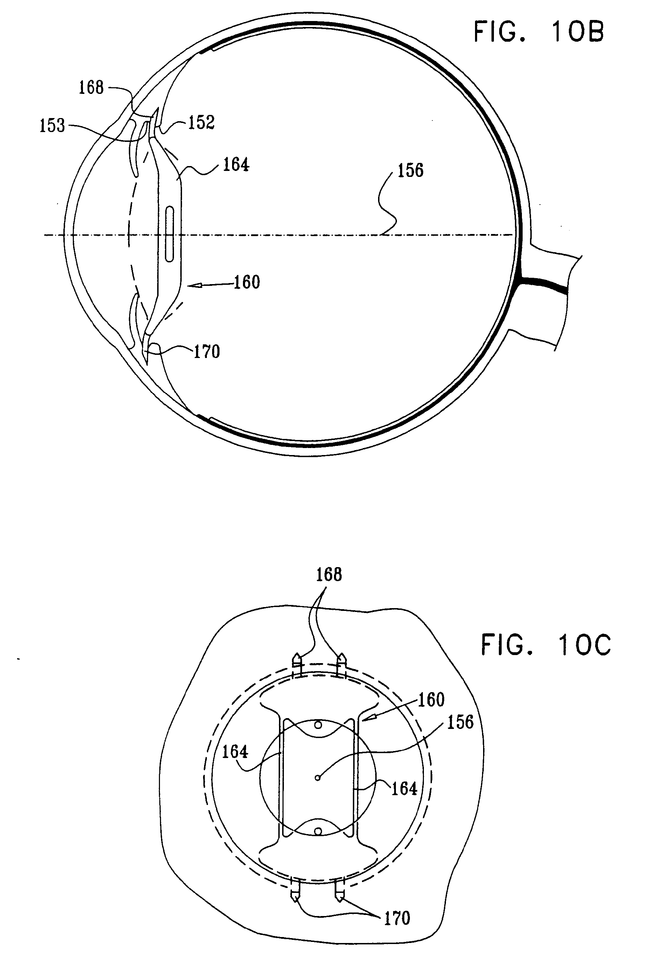

Reference is now made to Figs. 10A, 10B & 10C, which are respective pictorial, side sectional and front view illustrations of support element 160 following the insertion of second anchor portions 170 into the eye and the return of resilient side portions 164 to their original shape. As seen in Figs. 10A, 10B and 10C, side portions 164 of support element 160 return to their original shape and push downward on second anchor portions 170 until second anchor portions 170 engage the ciliary sulcus 153.

It is appreciated that first anchor portions 168 and second anchor portions 170 hold support element 160 firmly in place in the eye. It is also appreciated that support element 160 may be centered about axis 156 to enable centered positioning of an intraocular device to be implanted as described further hereinbelow. It is further appreciated that, as seen in Figs. 10A-10C, guides 150 may have been removed from the eye subsequent to the engagement of second anchor portions 170 with the ciliary sulcus 153. Alternatively, guides 150 may be obviated and support element 160 may be inserted in a fashion similar to that described hereinabove with reference to Figs. 9A-9C.

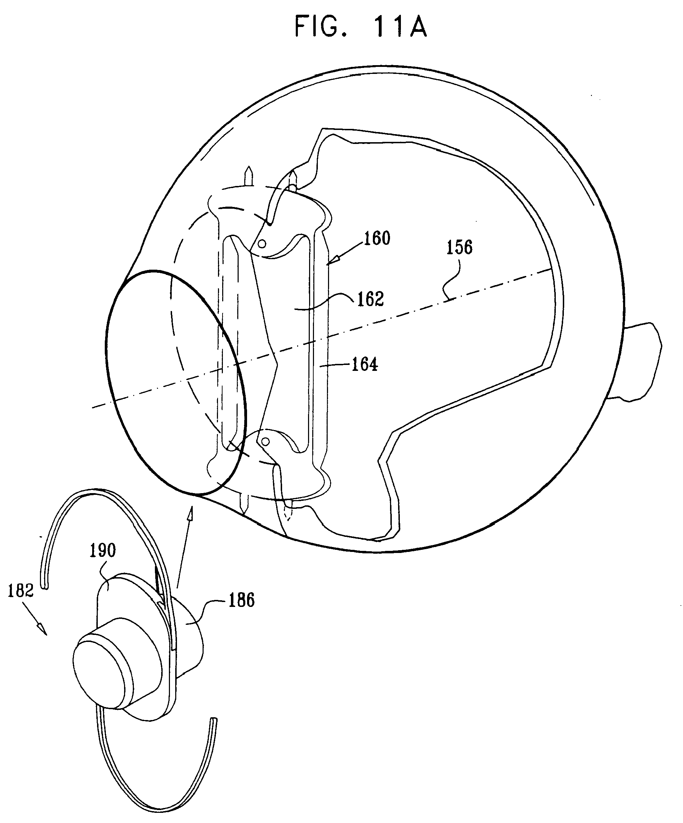

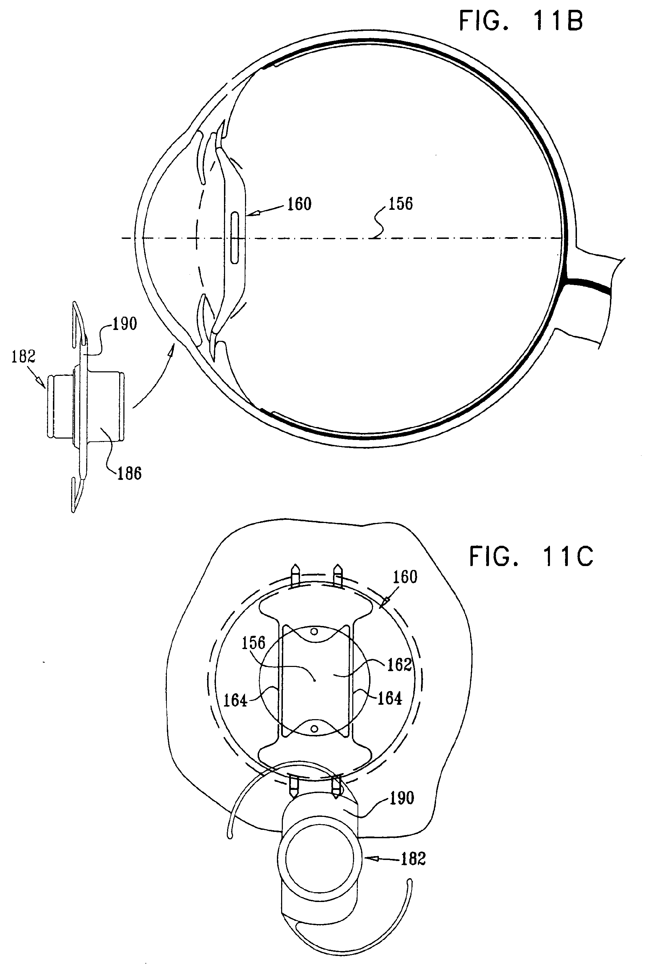

Reference is now made to Figs. 11A, 11B & 11C, which are respective pictorial, side sectional and front view illustrations of the insertion of a suitable intraocular optical device into optics receiving opening 162 of support element 160 of Figs. 8A-10C. As seen in Figs. 11A-11C, an intraocular optical device 182 is preferably inserted into support element 160 in an orientation centered about axis 156 and thereby retained against undesired axial motion along axis 156, particularly motion towards the rear of the eye. It will be appreciated that intraocular optical device 182 may be any suitable intraocular device, such as an intraocular lens or any device described in any of the U.S. Patents or Published U.S. Patent applications listed hereinabove in the Background of the Invention, the disclosures of which are hereby incorporated by reference. Optics receiving opening 162 of support element 160 preferably allows a generally elongate portion 186 of intraocular optical device 182 to extend therethrough, while side portions 164 of support element 160 preferably prevent a generally planar portion 190 of the device 182 from extending therebeyond.

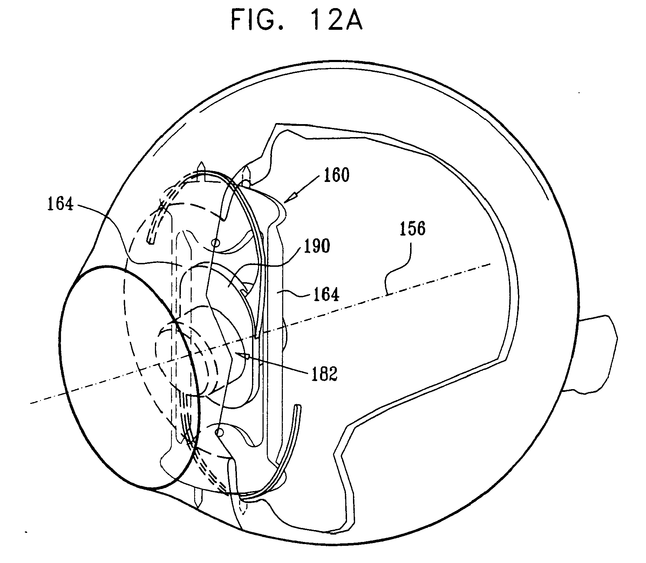

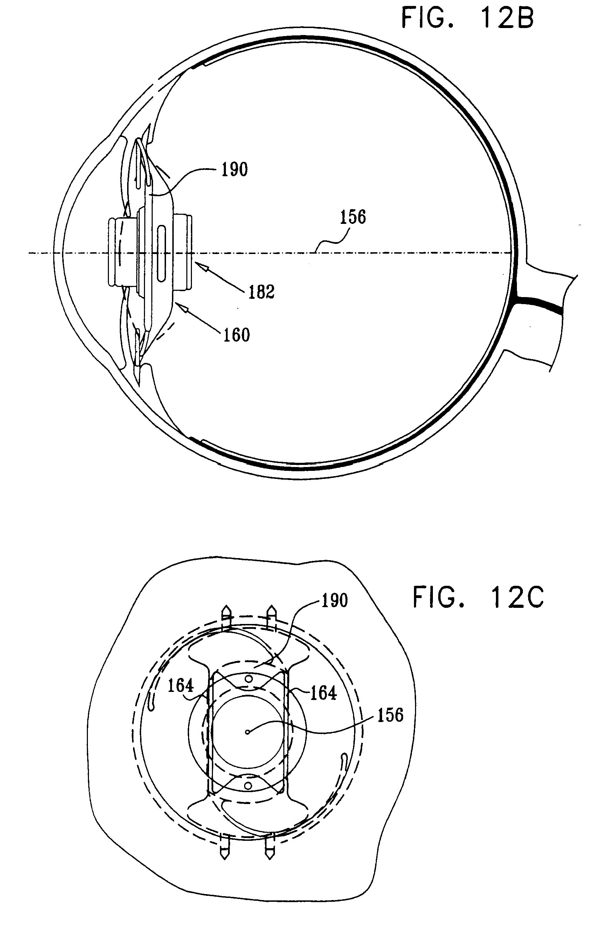

Reference is now made to Figs. 12A, 12B & 12C, which illustrate the intraocular optical device 182 implanted within the eye and being centered and retained against undesired rearward movement along axis 156 by engagement of planar portion 190 thereof with side portions 164 of support element 160.

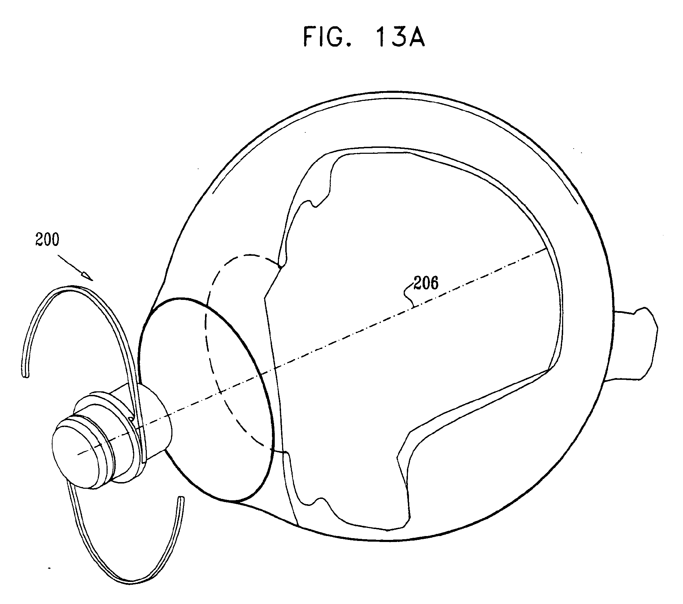

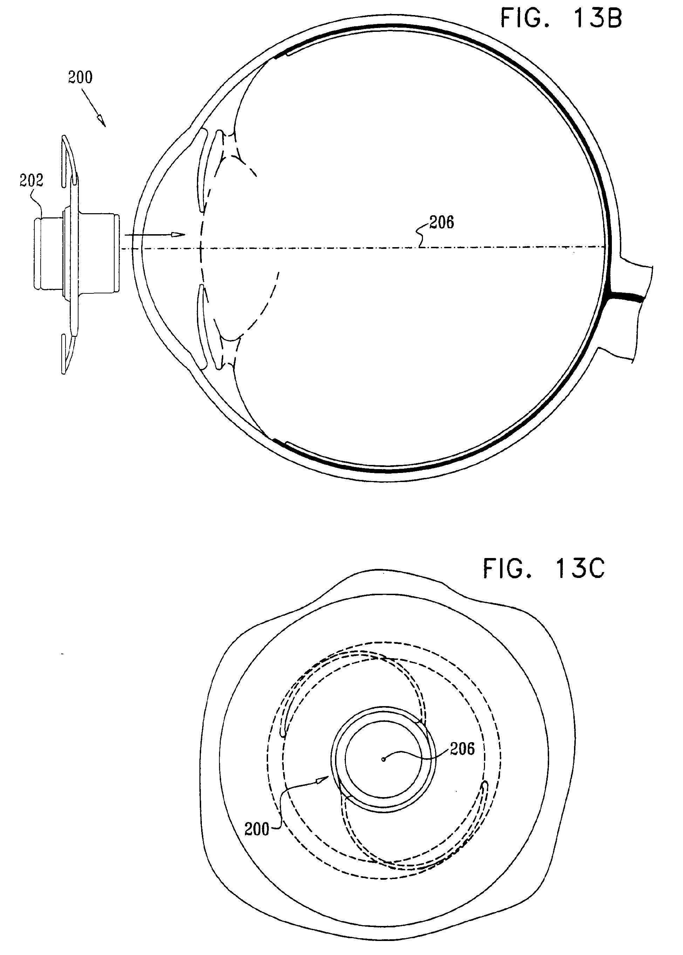

Reference is now made to Figs. 13A, 13B & 13C, which are respective pictorial, side sectional and front view illustrations of a first stage of implantation of an implantable intraocular device in accordance with yet another preferred embodiment of the present invention. In the embodiment of Figs. 13A - 13C, an intraocular optical device 200 is provided. Intraocular optical device 200 may be similar or identical to device 142 (Figs. 5A - 6C) and may be any suitable intraocular optical device, such as devices described in any of the U.S. Patents or Published U.S. Patent applications listed hereinabove in the Background of the Invention, the disclosures of which are hereby incorporated by reference, but is configured to provide an engagement portion, such as a groove 202, at a forward end thereof.

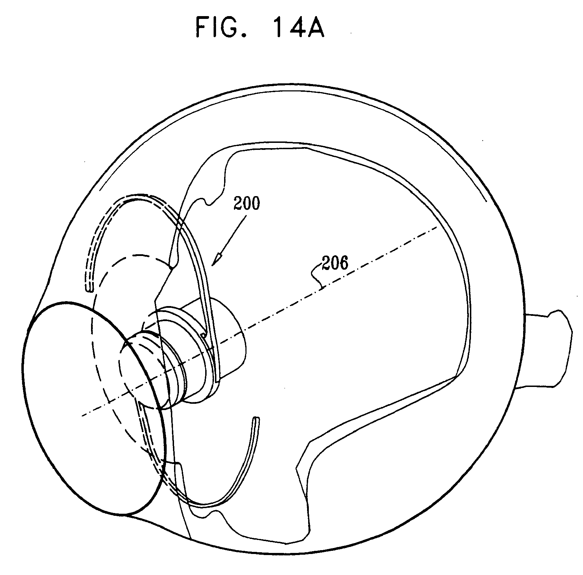

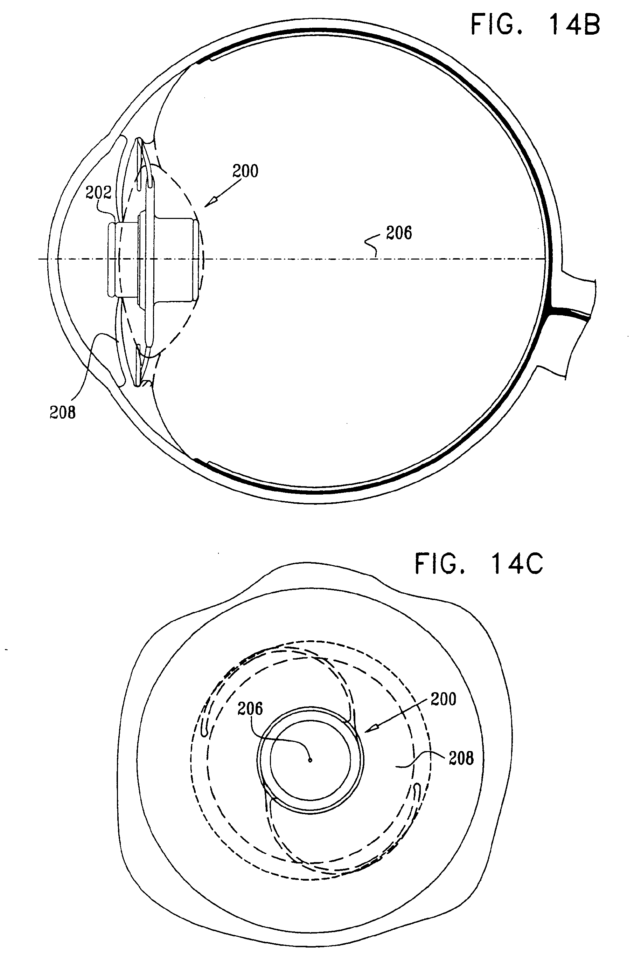

Reference is now made to Figs. 14A, 14B & 14C, which are respective pictorial, side sectional and front view illustrations of the intraocular optical device 200 of Figs. 13A-13C inserted into the posterior chamber of the eye in a conventional manner. Intraocular optical device 200 is generally centered about a central axis 206 defined by the iris 208.

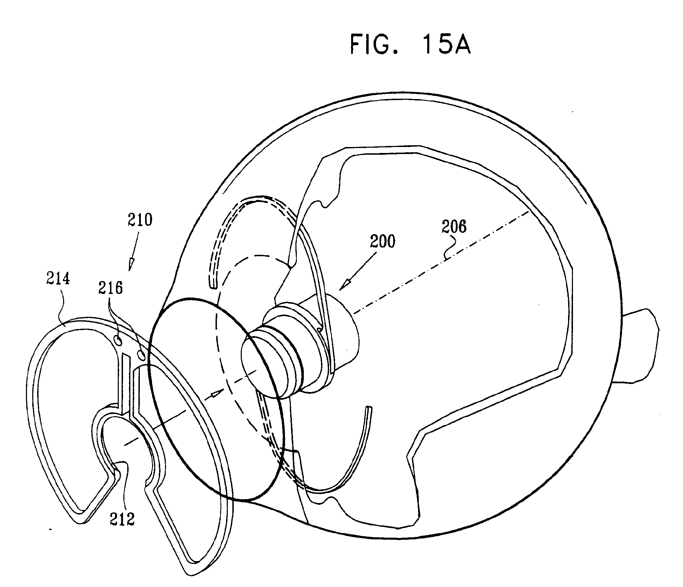

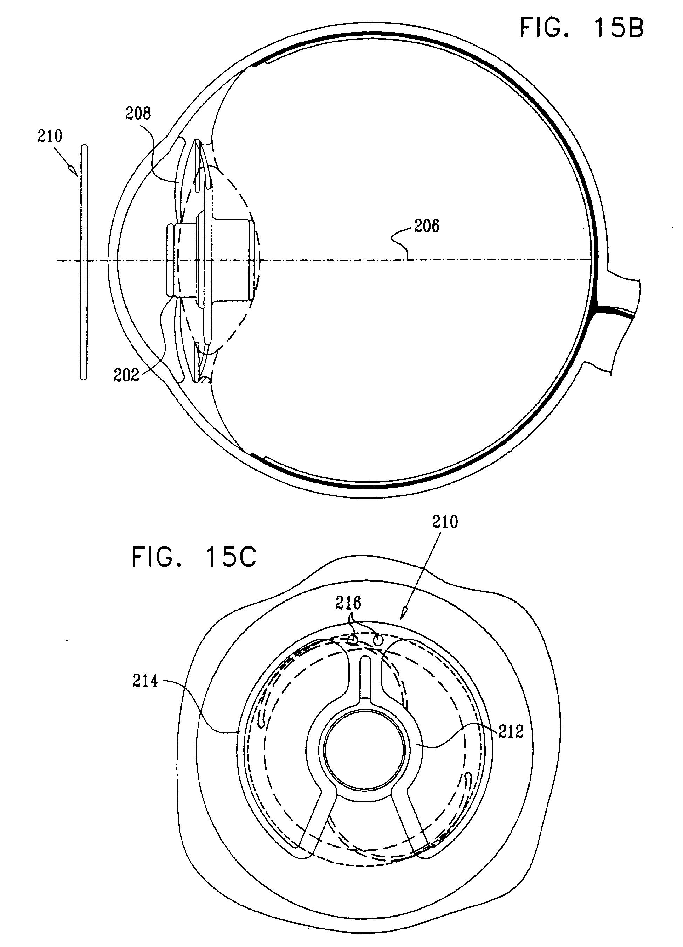

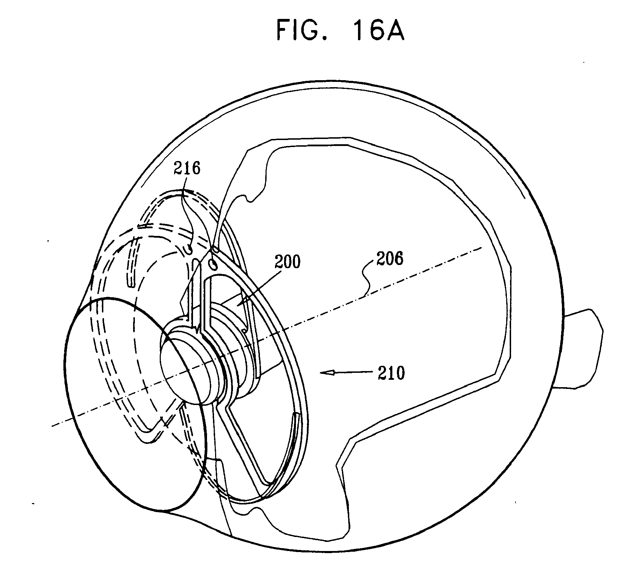

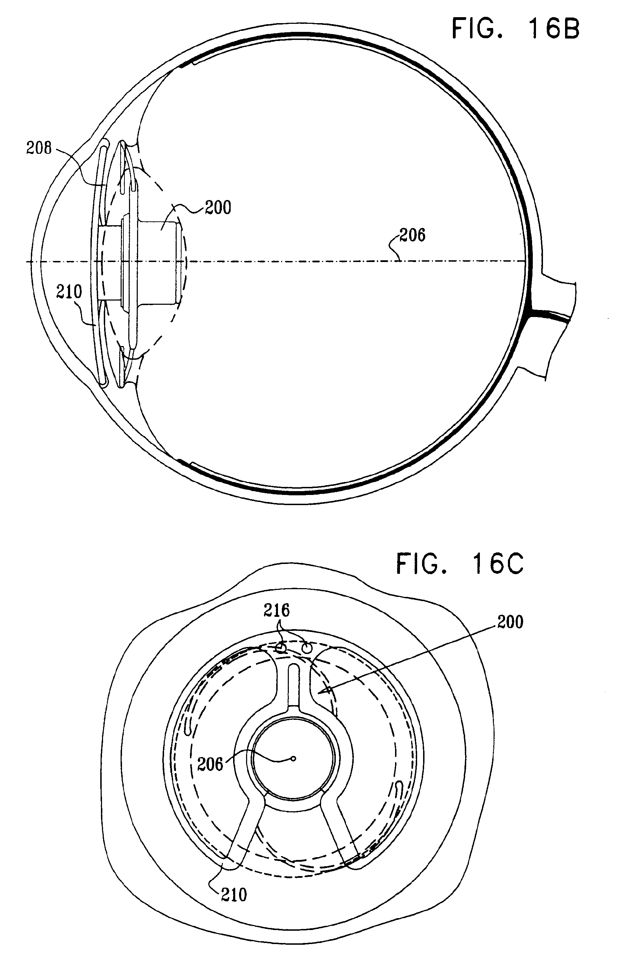

Reference is now made to Figs. 15A, 15B & 15C, which illustrate a retaining clip prepared for placement within the anterior chamber of the eye, and to Figs. 16A, 16B & 16C, which illustrate the retaining clip of Figs. 15A-15C following placement into the anterior chamber of the eye in engagement with the intraocular optical device of Figs. 13A-14C.

As seen in Figs. 15A-15C, a retaining clip 210 is preferably integrally formed of plastic, and includes an inner ring 212 adapted to engage groove 202 of intraocular optical device 200, preferably in a snap-fit engagement. Retaining clip 210 also includes an outer ring 214, which is adapted to lie in front of the iris 208 so as to be retained by the iris 208 against undesired axial movement along axis 206. Retaining clip 210 also includes hook insertion apertures 216 to facilitate engagement of retaining clip 210 and intraocular optical device 200.

As seen in Figs. 16A-16C, retaining clip 210 is in engagement with intraocular optical device 200 such that iris 208 lies between intraocular optical device 200 and retaining clip 210, causing retaining clip 210 and intraocular optical device 200 to be retained by the iris against undesired axial movement along axis 206. It is appreciated that retaining clip 210 may be manipulated, using at least one hook (not shown) inserted into at least one of hook insertion apertures 216, to facilitate engagement of inner ring 212 of retaining clip 210 and groove 202 of intraocular optical device 200.

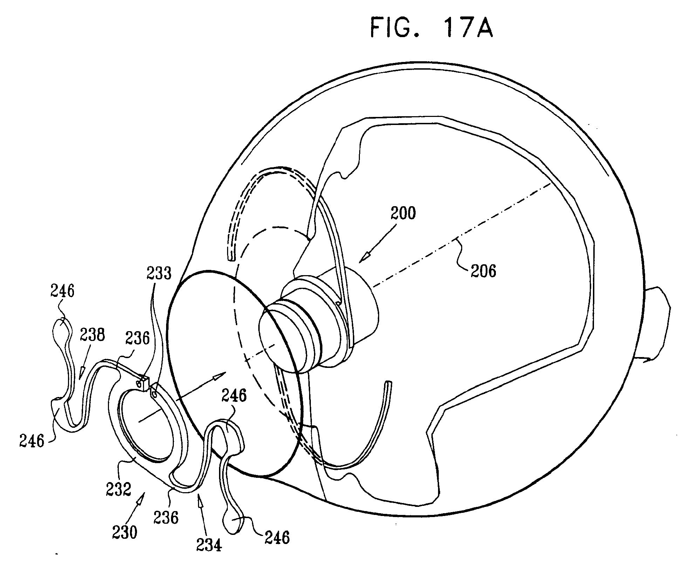

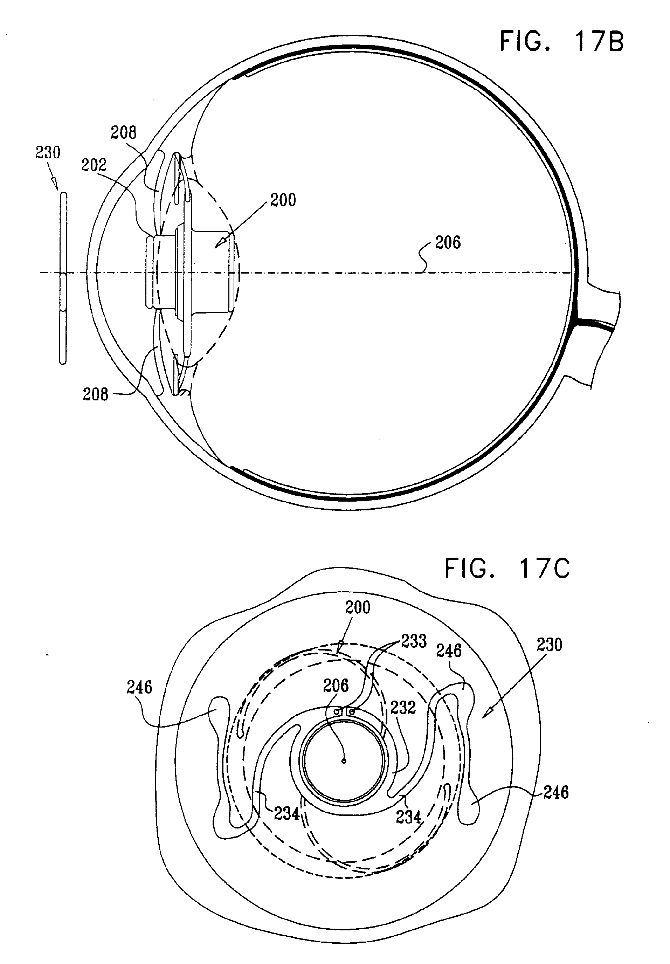

Reference is now made to Figs. 17A, 17B & 17C, which illustrate another embodiment of a retaining clip prepared for placement within the anterior chamber of the eye.

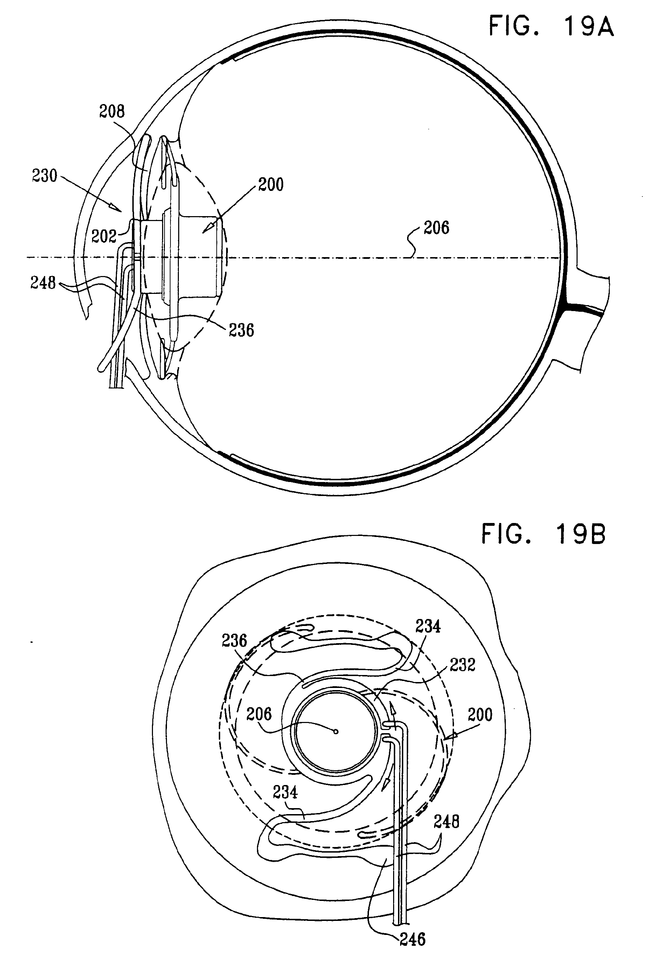

As seen in Figs. 17A, 17B & 17C, a retaining clip 230 is prepared for placement within the anterior chamber of the eye in engagement with intraocular optical device 200 of Figs. 13A-14C. As seen particularly in Fig. 17A, retaining clip 230 is preferably integrally formed of plastic and includes an inner ring 232, which is adapted to engage groove 202 of intraocular optical device 200, preferably in snap-fit engagement. Inner ring 232 includes hook insertion apertures 233 to facilitate engagement of retaining clip 230 and intraocular optical device 200, as described hereinbelow with reference to Figs. 19A-19C.

Retaining clip 230 preferably also includes a pair of outwardly extending arms 234 positioned at generally opposite sides of inner ring 232. Each of arms 234 preferably includes a generally straight portion 236 and a generally curved portion 238. Generally curved portion 238 is typically a generally "U" shaped portion and preferably includes at least one broadened support portion 246.

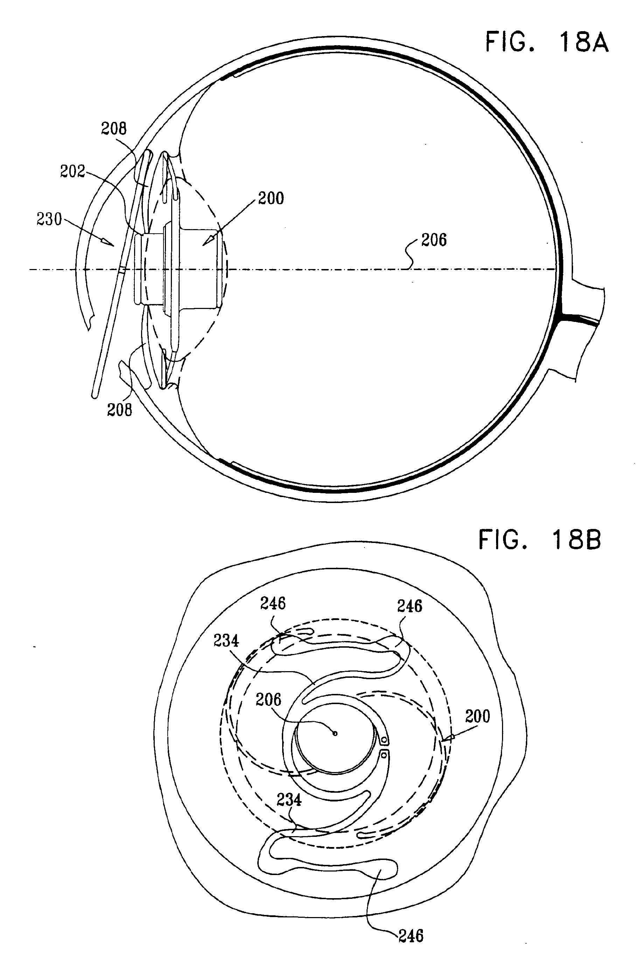

It is appreciated that, during insertion into the eye, retaining clip 230 is compressed by pushing arms 234 inwardly, thereby causing generally straight portions 236 to deform inwardly toward inner ring 232. The compression enables retaining clip 230 to be inserted into the eye without increasing the opening in the eye used to insert intraocular device 200, as described hereinbelow with reference to Figs. 18A-20B.

Reference is now made to Figs. 18A and 18B, which illustrate a first step in the insertion of the retaining clip of Figs. 17A-17C into the anterior chamber of the eye in engagement with the intraocular optical device of Figs. 13A-14C.

As seen in Figs. 18A and 18B, a first arm of the pair of arms 234 is inserted into the eye to lie in front of iris 208. As further seen in Figs. 18A and 18B, broadened support portions 246 of the first arm 234 are preferably inserted into the anterior chamber between the iris 208 and the cornea.

Reference is now made to Figs. 19A and 19B, which illustrate a further step in the insertion of the retaining clip of Figs. 17A-17C into the anterior chamber of the eye in engagement with the intraocular optical device of Figs. 13A-14C.

As seen in Figs. 19A and 19B, engagement of inner ring 232 with groove 202 of intraocular optical device 200 is facilitated by insertion of hooks 248 into hook insertion apertures 233 (Fig. 17C). Hooks 248 allow for the manipulation of inner ring 232 by increasing and decreasing the distance between hook insertion apertures 233, to ensure proper engagement with groove 202.

It is appreciated that the engagement of inner ring 232 with groove 202 causes generally straight portion 236 of first arm 234 to deform inwardly toward inner ring 232, as seen particularly in Fig. 19B. It is also appreciated that the engagement of inner ring 232 with groove 202 causes generally straight portion 236 of a second arm of pair of arms 234 to deform outwardly toward the cornea, as seen particularly in Fig. 19A.

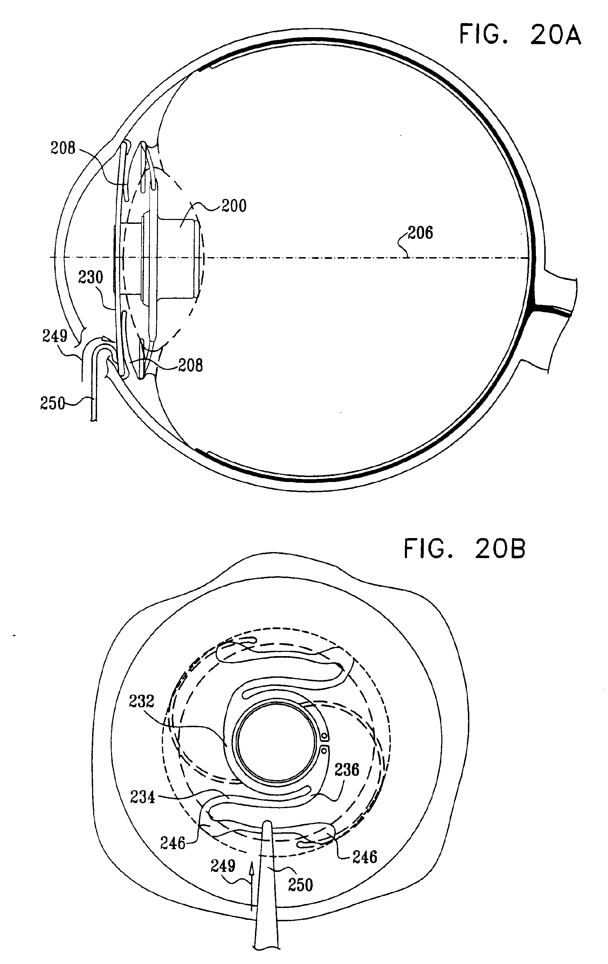

Reference is now made to Figs. 20A and 20B, which illustrate a final step in the insertion of the retaining clip of Figs. 17A-17C into the anterior chamber of the eye in engagement with the intraocular optical device of Figs. 13A-14C.

As seen in Figs. 20A and 20B, second arm 234 is compressed inwardly in the direction of arrow 249 by a hook 250, causing generally straight portion 236 of second arm 234 to deform inwardly toward inner ring 232 and to allow second arm 234 to be inserted into the anterior chamber of the eye between the iris 208 and the cornea.

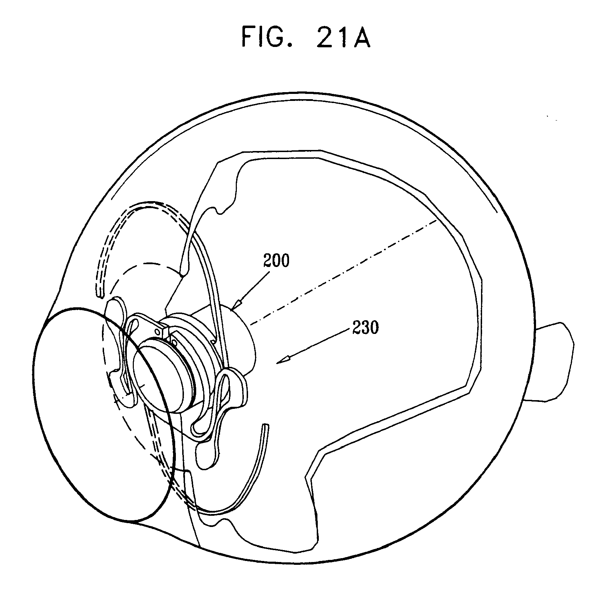

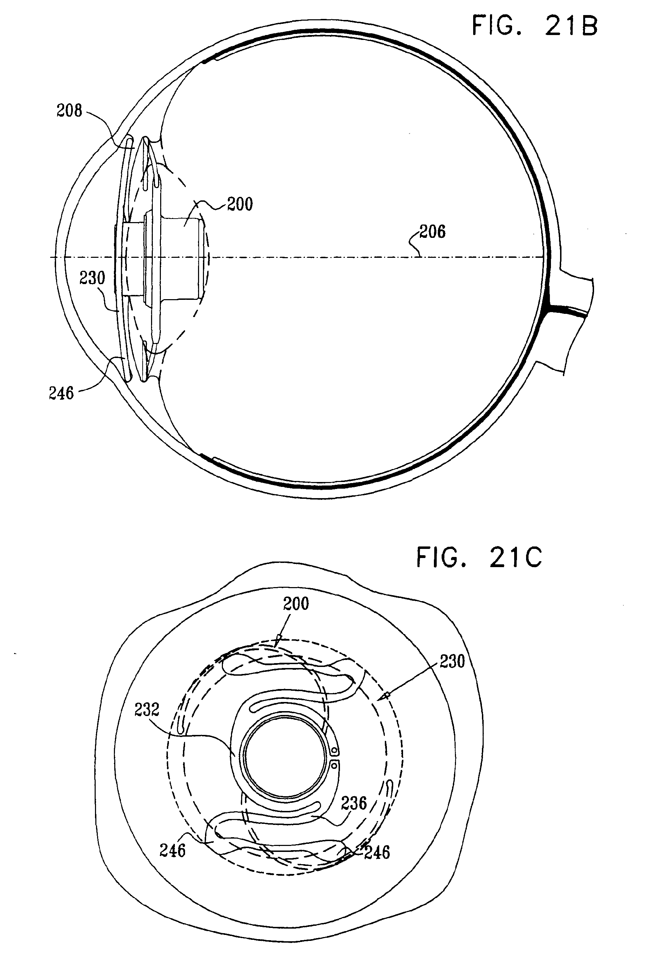

Reference is now made to Figs. 21A, 21B & 21C, which illustrate the retaining clip of Figs. 17A-20B following placement into the anterior chamber of the eye in engagement with the intraocular optical device of Figs. 13A-14C.

Figs. 21A, 21 B & 21C show retaining clip 230 of Figs. 17A-20B in its operative position in the anterior chamber of the eye, engaged with intraocular optical device 200.

As seen particularly in Fig. 21B, retaining clip 230 is located within the anterior chamber of the eye such that the iris 208 lies between intraocular optical device 200 and retaining clip 230, causing retaining clip 230 and intraocular optical device 200 to be retained by the iris against undesired axial movement along axis 206. As seen further in Fig. 21B, arms 234 are preferably formed so that support surfaces 246 will be located within the canal between the iris and the cornea when retaining clip 230 is centered about axis 206, thus restricting radial movement of retaining clip 230 about axis 206. As seen particularly in Fig. 21C, the broadened support portions 246 are preferably radially equidistant from the center of inner ring 232.

It is appreciated that the inward deforming of generally straight portions 236 provides tension which causes broadened support portions 246 of retaining clip 230 to remain firmly in place within the eye following insertion.

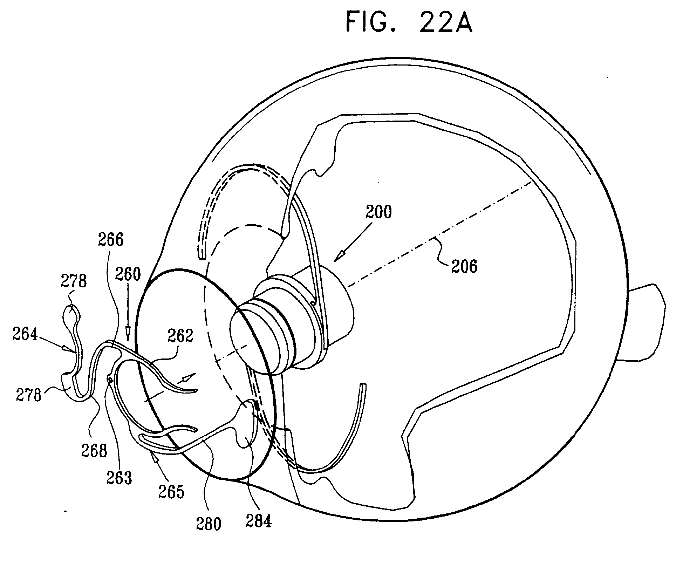

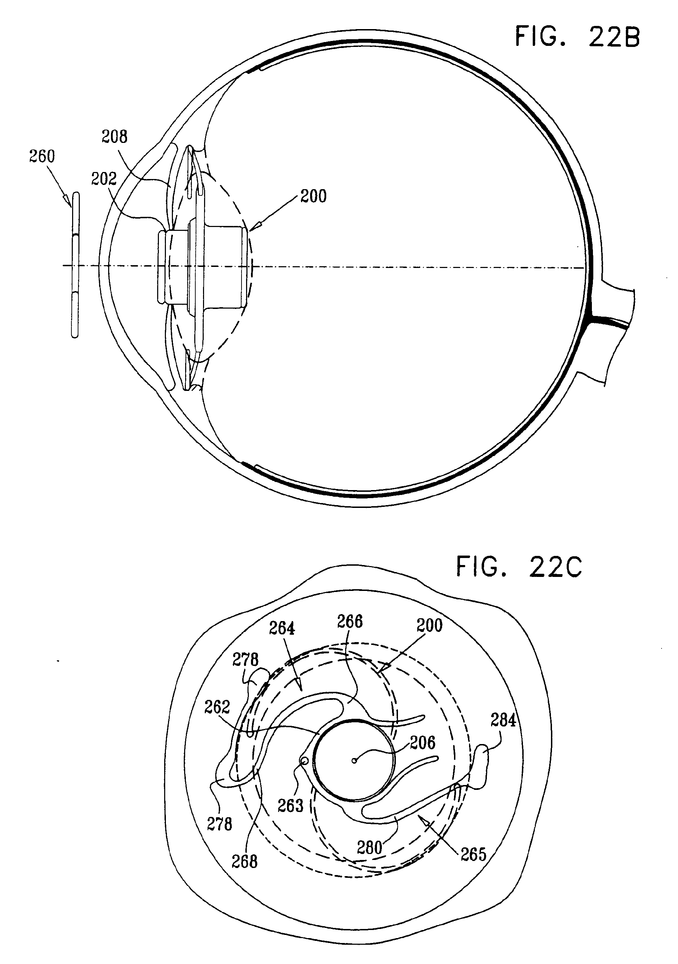

Reference is now made to Figs. 22A, 22B & 22C, which illustrate yet another embodiment of a retaining clip prepared for placement within the anterior chamber of the eye.

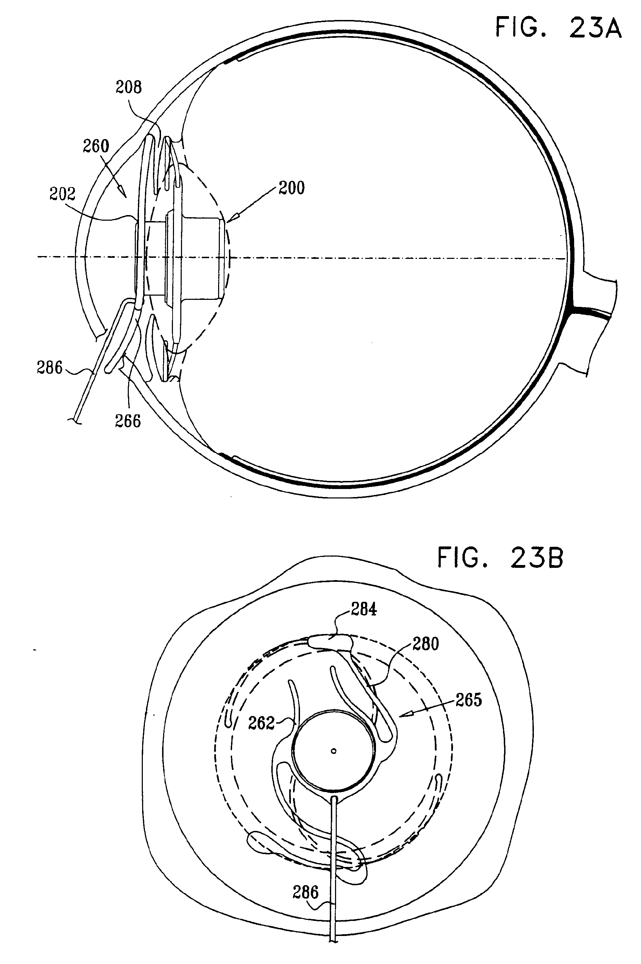

As seen in Figs. 22A, 22B & 22C, a retaining clip 260 is prepared for placement within the anterior chamber of the eye in engagement with intraocular optical device 200 of Figs. 13A-14C. As seen particularly in Fig. 22A, retaining clip 260 is preferably integrally formed of plastic and includes a generally circular optics engagement portion 262, which is adapted to engage groove 202 of intraocular optical device 200, preferably in a snap-fit engagement. Retaining clip 260 also preferably includes a hook insertion aperture 263 adjacent optics engagement portion 262 to facilitate engagement of retaining clip 260 and intraocular optical device 200, as described hereinbelow with reference to Figs. 23A and 23B.

Retaining clip 260 preferably also includes a first arm 264 and a second arm 265, which extend outwardly from generally circular engagement portion 262. First arm 264 preferably includes a generally straight portion 266 and a generally curved portion 268. Generally curved portion 268 is typically a generally "U" shaped portion and preferably includes at least one broadened support portion 278. Second arm 265 preferably includes an elongate section 280 terminating in a broadened support portion 284.

It is appreciated that, during insertion into the eye, retaining clip 260 is compressed by pushing arms 264 and 265 inwardly, thereby causing generally straight portion 266 and elongate section 280 to deform inwardly toward generally circular optics engagement portion 262. The compression enables retaining clip 260 to be inserted into the eye without increasing the opening in the eye used to insert intraocular device 200, as described hereinbelow with reference to Figs. 23A-24B.

Reference is now made to Figs. 23A and 23B, which illustrate a first step in the insertion of the retaining clip of Figs. 22A-22C into the anterior chamber of the eye in engagement with the intraocular optical device of Figs. 13A-14C.

As seen in Figs. 23A and 23B, retaining clip 260 is inserted into the eye into engagement with groove 202 of intraocular optical device 200 by manipulating optics engagement portion 262 using a hook 286 inserted into hook insertion aperture 263 (Fig. 22C). It is appreciated that optics engagement portion 262 is flexible to ensure proper engagement with groove 202.

It is appreciated that the engagement of optics engagement portion 262 with groove 202 also enables placement of second arm 265 into position in the anterior chamber between the iris 208 and the cornea. It is also appreciated that the engagement of optics engagement portion 262 with groove 202 causes elongate section 280 of second arm 265 to deform inwardly toward optics engagement portion 262, as seen particularly in Fig. 23B. It is further appreciated that the engagement of optics engagement portion 262 with groove 202 causes generally straight portion 266 of first arm 264 to deform outwardly toward the cornea, as seen particularly in Fig. 23A.

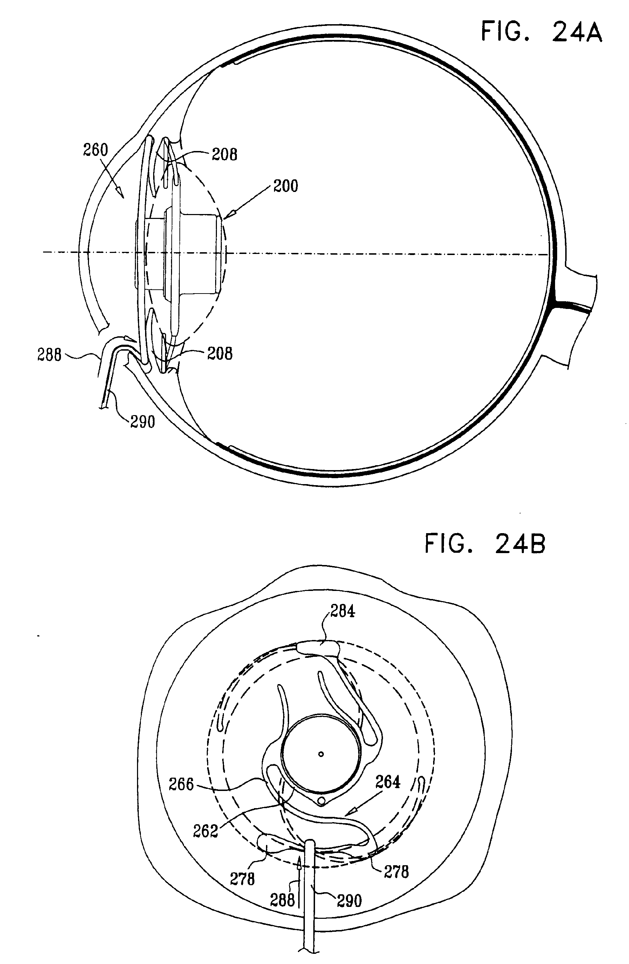

Reference is now made to Figs. 24A and 24B, which illustrate a further step in the insertion of the retaining clip of Figs. 22A-22C into the anterior chamber of the eye in engagement with the intraocular optical device of Figs. 13A-14C.

As seen in Figs. 24A and 24B, first arm 264 is compressed inwardly in the direction of arrow 288 by a hook 290, causing generally straight portion 266 of first arm 264 to deform inwardly toward optics engagement portion 262 and to allow first arm 264 to be inserted into the anterior chamber of the eye between the iris 208 and the cornea.

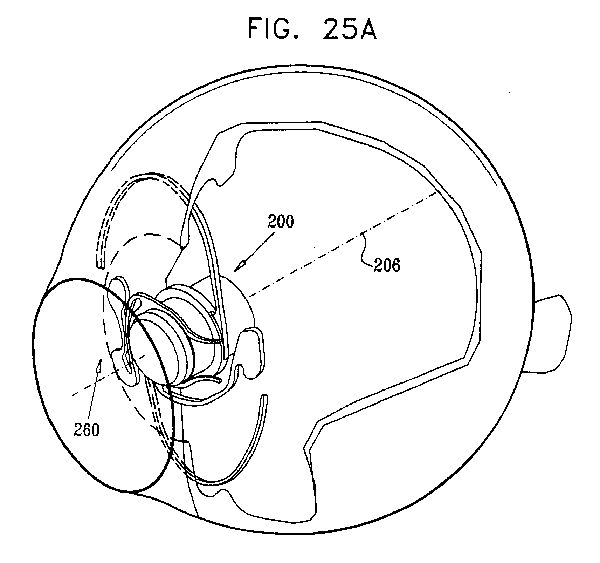

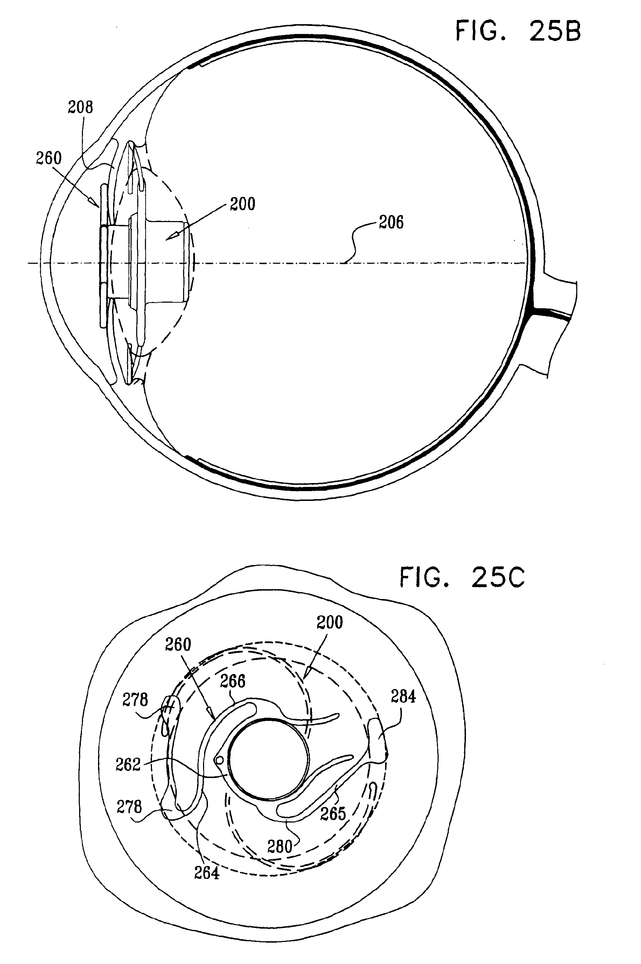

Reference is now made to Figs. 25A, 25B & 25C, which illustrate the retaining clip of Figs. 22A-24B following placement into the anterior chamber of the eye in engagement with the intraocular optical device of Figs. 13A-14C.

Figs. 25A, 25B & 25C show retaining clip 260 of Figs. 22A-24B in its operative position in the anterior chamber of the eye, engaged with intraocular optical device 200.

As seen particularly in Fig. 25B, retaining clip 260 is located within the anterior chamber of the eye such that the iris 208 lies between intraocular optical device 200 and retaining clip 260, causing retaining clip 260 and intraocular optical device 200 to be retained by the iris against undesired axial movement along axis 206. First and second arms 264 and 265 are preferably formed so that broadened support portions 278 and 284 will be located within the canal between the iris and the cornea when retaining clip 260 is centered about axis 206, thus restricting radial movement of retaining clip 260 about axis 206. As seen particularly in Fig. 25C, broadened support portions 278 and 284 are radially equidistant from the center of generally circular engagement portion 262 and thus from axis 206, when retaining clip 260 is centered about axis 206.

It is appreciated that the inward deforming of generally straight portion 266 and elongate section 280 provides tension which causes broadened support portions 278 and 284 of retaining clip 260 to remain firmly in place within the eye following insertion.

It will be appreciated by persons skilled in the art that the present invention is not limited by what has been particularly shown and described hereinabove. Rather the scope of the present invention includes both combinations and subcombinations of the various features described hereinabove as well as variations and modifications which would occur to persons skilled in the art upon reading the specification and which are not in the prior art.

## Claims

1. An intraocular device comprising:
at least one lens; and
a flexible integrally formed support element for retaining said at least one lens, said support element comprising first and second axially separated anchor portions which are joined by at least one flexible joining element which supports said at least one lens without having to be fixed thereto.

2. An intraocular device according to claim 1 and wherein said support element is adapted to be anchored to an eye at a ciliary sulcus thereof by engagement of said first and second anchor portions with said ciliary sulcus.

3. An intraocular device according to claim 1 and wherein said support element is operative to retain said at least one lens from undesired axial motion along a central axis defined by an iris of an eye.

4. An intraocular device comprising:
at least one optical lens; and
at least one flexible elongate support element arranged to support said at least one optical lens within an eye, without being fixed to said at least one optical lens.

5. An intraocular device according to claim 4 and wherein said at least one flexible elongate support element is adapted to be anchored to said eye at one of a ciliary body and a ciliary sulcus thereof.

6. An intraocular device according to claim 4 and wherein said at least one flexible elongate support element comprises multiple flexible elongate support elements defining a retaining structure.

7. An intraocular device according to claim 4 and wherein said at least one flexible elongate support element is operative to retain said at least one optical lens against undesired motion toward a rear of said eye.

8. An intraocular device according to claim 4 and wherein said at least one flexible elongate support element is operative to retain said at least one optical lens from undesired axial motion along a central axis defined by an iris of said eye.

9. An intraocular device according to claim 4 and wherein said at least one flexible elongate support element is operative to retain said at least one optical lens from undesired radial motion relative to a central axis defined by an iris of said eye.

10. An intraocular device according to claim 4 and wherein said at least one optical lens has haptics fixed thereto.

11. An intraocular device comprising:
at least one optical lens; and
an anterior chamber locatable lens retainer removably engageable with said at least one optical lens when located in an anterior chamber of an eye for restricting generally axial displacement of said at least one optical lens in a rearward direction away from said anterior chamber.

12. An intraocular device according to claim 11 and wherein said at least one optical lens has haptics fixed thereto.

13. An intraocular device according to claim 11 and wherein said anterior chamber locatable lens retainer engages said optical lens in a snap-fit type engagement.

14. An intraocular device according to claim 11 and wherein said anterior chamber locatable lens retainer includes an inner portion for engaging said optical lens and an outer portion for retaining said anterior chamber locatable lens retainer against an iris of an eye, preventing said anterior chamber locatable lens retainer from motion rearward of said iris.

15. An intraocular device according to claim 11 and wherein said lens retainer includes at least one broadened support portion.

16. An intraocular device according to claim 15 and wherein said at least one broadened support portion is adapted to be seated within a canal between said iris and a cornea of said eye.

17. An intraocular device support comprising:
an integrally formed support element having first and second axially separated anchor portions which are joined by at least one flexible joining element which allows the axial separation of said first and second axially separated anchor portions to be varied by axial compression thereof.

18. An intraocular device support according to claim 17 and wherein said support element is adapted to be anchored to an eye at a ciliary sulcus thereof by engagement of said first and second anchor portions with said ciliary sulcus.
